(19) Europäisches Patentamt
European Patent Office
Office européen des brevets

(11) Publication number: **0 058 918**
**B1**

(12) **EUROPEAN PATENT SPECIFICATION**

(45) Date of publication of patent specification: **29.01.86**

(21) Application number: **82101133.5**

(22) Date of filing: **16.02.82**

(51) Int. Cl.$^4$: **C 07 D 207/16,**
C 07 D 217/26,
C 07 D 277/06,
C 07 C 133/02,
C 07 D 207/12,
A 61 K 31/175, A 61 K 31/40,
A 61 K 31/47

(54) **Aza analogs of aminoacid derivatives as antihypertensives.**

(30) Priority: **17.02.81 US 235321**
**12.01.82 US 338035**

(43) Date of publication of application:
**01.09.82 Bulletin 82/35**

(45) Publication of the grant of the patent:
**29.01.86 Bulletin 86/05**

(84) Designated Contracting States:
**AT BE CH DE FR GB IT LI LU NL SE**

(56) References cited:
**EP-A-0 054 862**
**FR-A-2 269 521**
**US-A-4 217 130**

(73) Proprietor: **MERCK & CO. INC.**
**126, East Lincoln Avenue P.O. Box 2000**
**Rahway New Jersey 07065 (US)**

(72) Inventor: **Greenlee, William J.**
**115 Herrick Avenue**
**Teaneck New Jersey 07666 (US)**
Inventor: **Thorsett, Eugene D.**
**4 Poplar Place**
**Fanwood New Jersey 07023 (US)**

(74) Representative: **Abitz, Walter, Dr.-Ing. et al**
**Abitz, Morf, Gritschneder, Freiherr von Wittgenstein Postfach 86 01 09**
**D-8000 München 86 (DE)**

**The file contains technical information submitted after the application was filed and not included in this specification**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 058 918 B1

Courier Press, Leamington Spa, England.

**Description**

Background of Invention

The invention in its broad aspects relates to aza analogs of carboxyalkyl dipeptides derivatives which are useful as converting enzyme inhibitors and as antihypertensives. The compounds of this invention are represented by the following general formula:

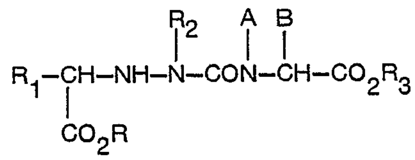 I

wherein:

R and $R_3$ are independently hydrogen, $C_{1-6}$ alkyl, ar-$C_{1-6}$-alkyl;

$R_1$ is aralkyl, heteroaralkyl, substituted aralkyl and substituted heteroaralkyl wherein the alkyl groups contain 1—6 carbon atoms and wherein the substituents in the aryl and heteroaryl groups can each independently be amino, halo, amino-$C_{1-6}$-alkyl, hydroxy, $C_{1-6}$ alkoxy, or $C_{1-6}$ alkyl; alkyl of 1—8 carbon atoms and alkyl of 1—8 carbon atoms substituted by amino, acylamino wherein acyl represents $C_{1-6}$ alkanoyl or aroyl groups, heteroarylamino, aryloxy, heteroaryloxy, arylthio, heteroarylthio or hydroxyl;

$R_2$ is hydrogen, $C_{1-6}$alkyl, amino-$C_{1-6}$-alkyl, ar-$C_{1-6}$-alkyl;

A is cycloalkyl of 5—7 carbon atoms, aryl, heteroaryl;

B is hydrogen, $C_{1-6}$ alkyl; or

A and B can be joined together to form ring structures which include N—$CHCO_2R_3$ and which have the formulae

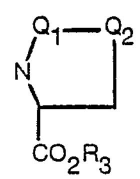

and

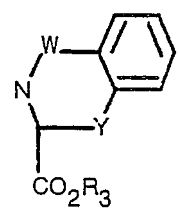

wherein

$R_3$ is as defined above;

$Q_1$ and $Q_2$, taken together, are $CH_2CH_2$, $CH_2$—S, $CHR_4S$, $CH_2$—CH—$OR_5$ wherein $R_4$ is aryl or aryl substituted by hydroxyl, amino, $C_{1-6}$ alkyl, amino, halo, or $C_{1-6}$ alkoxy; and

$R_5$ is hydrogen, $C_{1-6}$ alkyl, aryl, ar-$C_{1-6}$-alkyl, or $CONR_6R_7$ wherein $R_6$ and $R_7$ can each independently be hydrogen, $C_{1-6}$ alkyl, ar-$C_{1-6}$-alkyl;

W is a bond, $CH_2$;

Y is a bond $CH_2$, $CH_2CH_2$;

the term "aryl" representing phenyl, naphthyl or biphenyl; the term "heteroaryl" representing pyridyl, thienyl, furyl, indolyl, benzthienyl, imidazolyl and thiazolyl; and the terms "aralkyl" and "heteroaralkyl" representing the corresponding radicals having 1—6 carbon atoms in the alkyl portion; and, the pharmaceutically acceptable salts thereof.

Preferred are compounds of Formula I wherein:

R and $R_3$ are independently hydrogen, $C_{1-6}$alkyl, ar-$C_{1-6}$-alkyl;

$R_1$ is aralkyl and heteroaralkyl wherein the alkyl groups contain 1—4 carbon atoms; substituted aralkyl, and substituted heteroaralkyl wherein the alkyl groups contain 1—4 carbon atoms and the substituents are halo, amino, amino $C_{1-6}$alkyl, hydroxy; substituted alkyl of 1—6 carbon atoms wherein the substituent is amino, acylamino, wherein aryloxy, aryl represents $C_{1-6}$ alkanoyl or aroyl groups, arylthio, heteroarylthio, or hydroxy;

$R_2$ is hydrogen, $C_{1-6}$alkyl, amino $C_{1-6}$alkyl;

A is cycloalkyl of 5—7 carbon atoms;

B is hydrogen; or,

A and B can be joined together to form ring structures which include $NCHCO_2R_3$ and which have the formula:

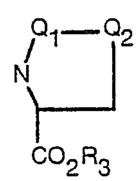

wherein:

$R_3$ is as defined above;

$Q_1$ and $Q_2$, taken together, are $CH_2CH_2$, $CH_2S$, $CHR_4S$, $CH_2CH—OR_5$ wherein $R_4$ is aryl or aryl substituted by hydroxyl; and, $R_5$ is $C_{1-6}$ alkyl, ar-$C_{1-6}$-alkyl.

More preferred are compounds of Formula I wherein:

R and $R_3$ are independently hydrogen, $C_{1-6}$alkyl, ar-$C_{1-6}$-alkyl;

$R_1$ is aralkyl and heteroaralkyl wherein the alkyl groups contain 1—4 carbon atoms; substituted aralkyl and substituted heteroaralkyl wherein the alkyl groups contain 1—4 carbon atoms and the substituents are halo or hydroxy; substituted alkyl of 1—6 carbon atoms wherein the substituent is aryloxy or arylthio;

$R_2$ is hydrogen, $C_{1-6}$alkyl, amino $C_{1-6}$alkyl;

A and B can be joined together to form ring structures which include $NCH—CO_2R_3$ and which have the formula:

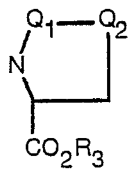

wherein:

$R_3$ is as defined above;

$Q_1$ and $Q_2$, taken together, are $CH_2CH_2$, $CH_2S$, $CHR_4S$, $CH_2CH—OR_5$ wherein $R_4$ is aryl or aryl substituted by hydroxyl; and $R_5$ is $C_{1-6}$alkyl.

Most preferred are compounds of Formula I wherein:

R is hydrogen, $C_{1-6}$alkyl;

$R_1$ is aralkyl and heteroaralkyl wherein the alkyl groups contain 1—3 carbon atoms; substituted aralkyl wherein the alkyl groups contain 1—3 carbon atoms and the substituents are halo or hydroxy; substituted alkyl of 1—6 carbon atoms wherein the substituent is aryloxy or arylthio;

$R_2$ is hydrogen, methyl, 4-aminobutyl;

$R_3$ is hydrogen;

A and B can be joined together to form ring structures which include $NCHCO_2R_3$ and which have the formula:

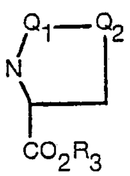

wherein $R_3$ is as defined above, and which are proline, thiaproline, or 4-carboxy-2-(2-hydroxyphenyl)thiazolidine,

The preferred, more preferred and most preferred compounds also include the pharmaceutically acceptable salts thereof.

Especially preferred compounds of the invention are:

N-(1-carboxy-3-phenylpropyl)-α-azaalanyl-L-proline;
N-[1(S)-carboxy-3-phenylpropyl]-α-azaalanyl-L-proline;
N-(1-ethoxycarbonyl-3-phenylpropyl]-α-azaalanyl-L-proline;
N-[1(S)-carboethoxy-3-phenylpropyl]-α-azaalanyl-L-proline;
N-(1-carboethoxy-3-phenylpropyl]-α-azaalanyl-L-proline ethyl ester;
N-[1(S)-carboethoxy-3-phenylpropyl]-α-azaalanyl-L-proline ethyl ester;
$N^{\alpha}$-(1-carboxy-3-phenylpropyl)-α-azalysyl-L-proline; and, ·
$N^{\alpha}$-[1(S)-carboxy-3-phenylpropyl]-α-azalysyl-L-proline.

The lower alkyl groups, except where noted otherwise, represented by any of the variables include straight or branched hydrocabon radicals of from one to six carbon atoms, for example, methyl, ethyl, propyl, isopropyl, butyl, isobutyl, t-butyl, pentyl, isopentyl, or hexyl. The aralkyl groups represented by any of the above variables have, except where noted otherwise, from one to six carbon atoms in the alkyl portion thereof and include for example, benzyl or phenethyl, Halo means chloro, bromo, iodo or fluoro. Aryl, where it appears in any of the radicals unless otherwise noted, represents phenyl, naphthyl or biphenyl. Heteroaryl groups, where they appear, represents pyridyl, thienyl, furyl, indolyl, benzthienyl, imidazoyl and thiazolyl. Acyl refers to $C_{1-6}$alkanoyl and aroyl groups.

The products of Formula (I) and the preferred subgroups can be produced by one or more of the methods and subroutes depicted in the following Reaction Schemes wherein R, $R_1$, $R_2$, $R_3$, A, B, W, and Y are as defined above unless stated otherwise:

REACTION SCHEME I

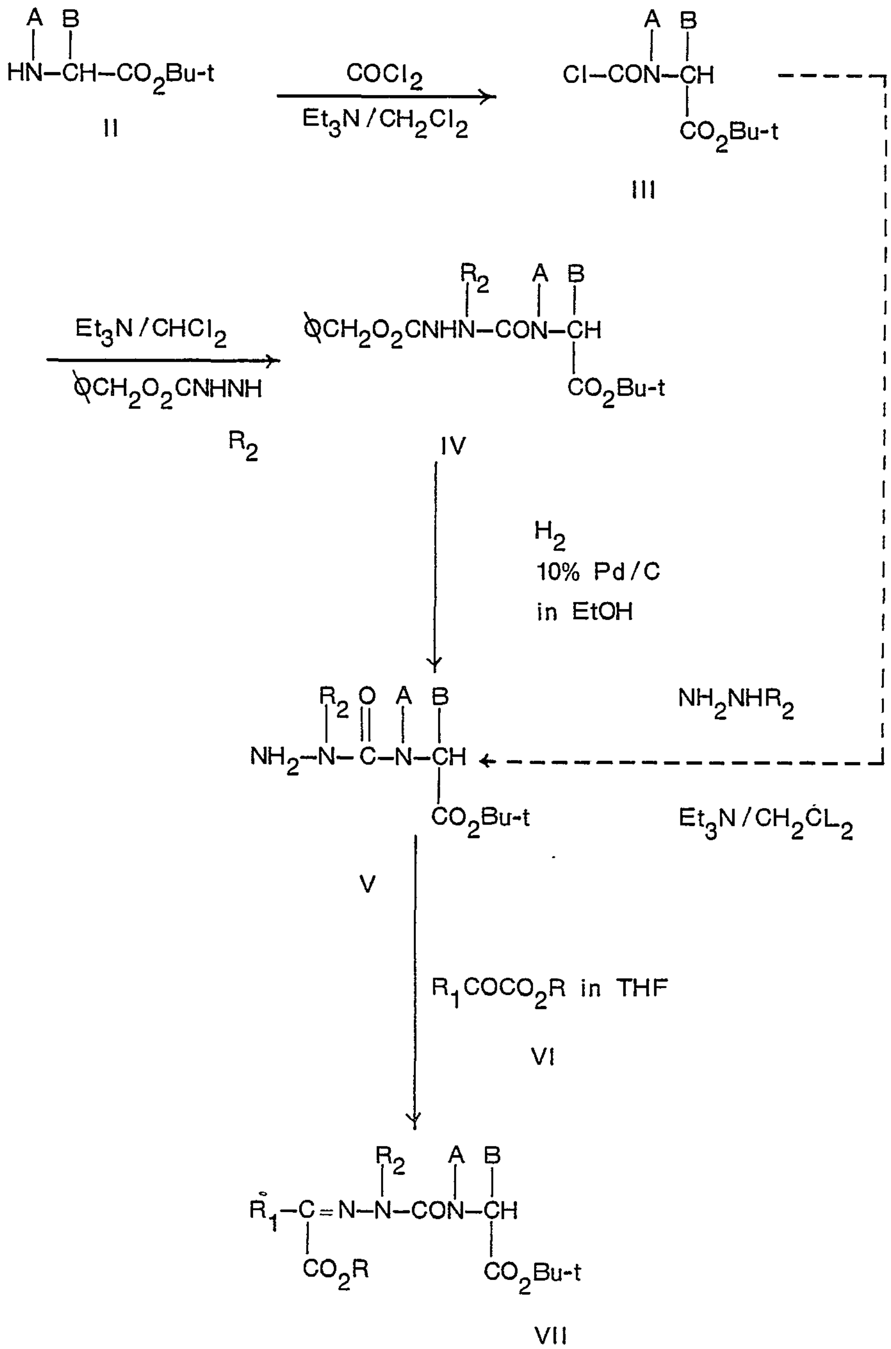

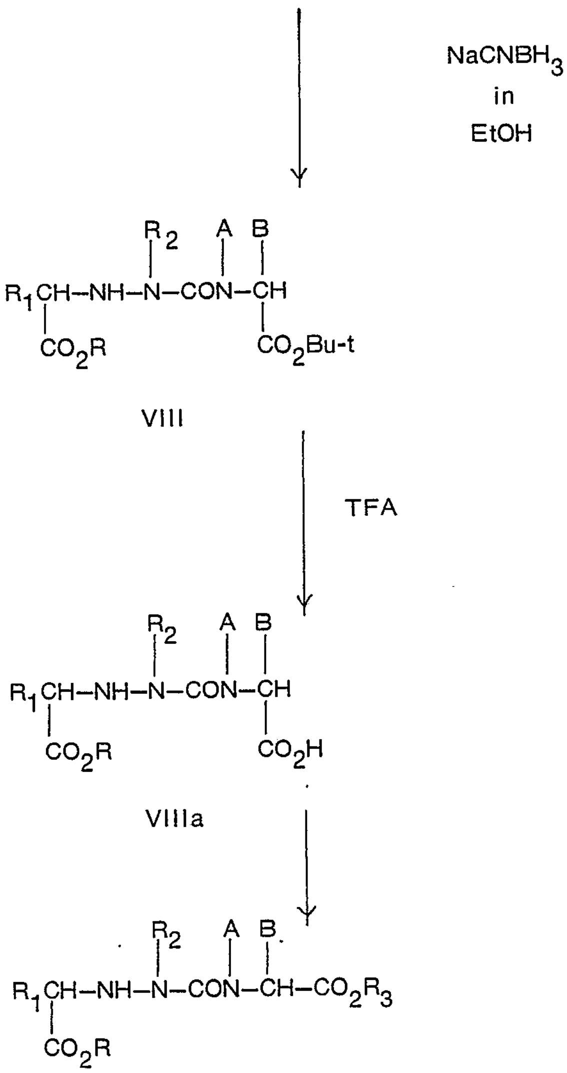


VIII

VIIIa

I

REACTION SCHEME II

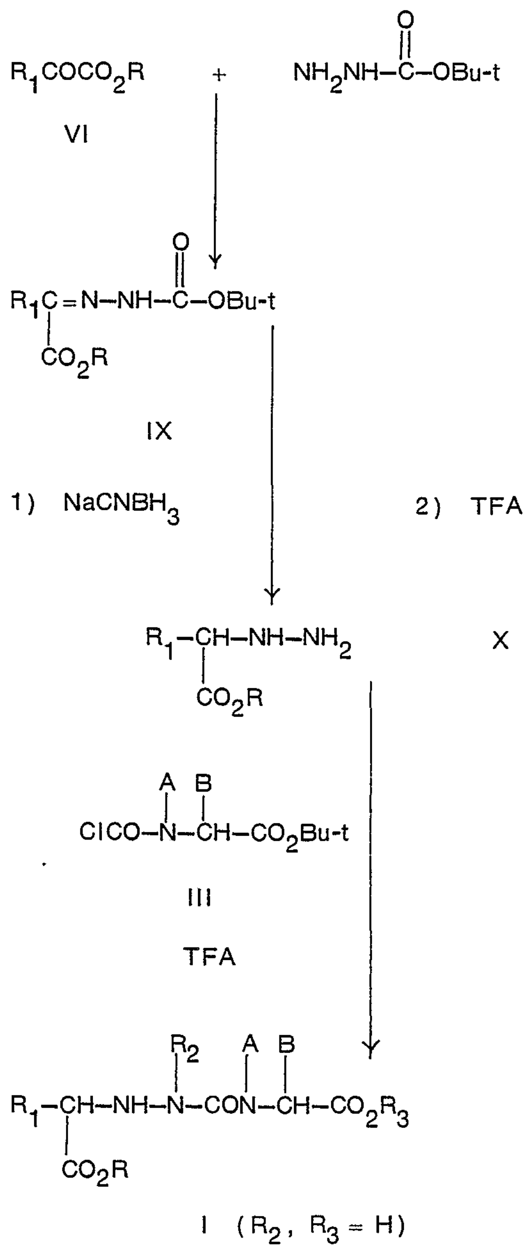


As depicted in Reaction Scheme I, the aza analog compounds of the invention are generally produced by reacting the t-butyl ester of an imino acid II with phosgene in the presence of triethyl amine in methylene chloride to obtain N-chlorocarbonyl derivative III. This intermediate III, in the presence of triethyl amine in methylene chloride, can then be reacted, for example, with isopropyl or N-isopropyl-N'-carbobenzyloxy hydrazine (prepared according to known methods such as that described by C. J. Gray, et al, *Tetrahedron, 33,* 837—40 (1977)] to obtain, for example by isolation, filtration and condensation, protected intermediate IV. Hydrogen, in the presence of a 10% palladium-carbon catalyst in ethanol, is reacted with IV to afford deprotected intermediate derivative V. Alternatively, III can be reacted with a substituted hydrazine in the presence of triethylamine in methylene chloride to afford V. Derivative V is dissolved in tetrahydrofuran (THF) and reacted with α-keto ester or acid VI to yield an α-keto ester-carbazone VII which, after treatment with sodium cyanoborohydride in ethanol, affords the α-aza-t-butyl ester peptide derivative VIII. Peptide derivative VIII is reacted with trifluoroacetic acid (TFA) to remove the t-butyl ester group yielding VIIIa. $R_3$, when it is not hydrogen, can be introduced using $R_3X$ (X=bromo, iodo) and a base such as cesium hydroxide. Removal of protecting groups, if present, then affords compounds of Formula I. Bis-esters of I can be obtained by Fisher esterification, for example, by treating Formula I compounds in an alcohol with dry HCl at room temperature.

Alternatively, compounds of Formula I when $R_2$=H can be obtained as shown in Reaction Scheme II. This procedure involves the synthesis of α-hydrazino acids or esters X followed by their condensation with N-chlorocarbonyl intermediates III. Subsequent esterification, if desired, and removal of protecting groups under standard conditions affords compounds of Formula I ($R_2$=H).

As will be evident to those skilled in the art and as demonstrated in the Examples which follow, reactive groups not involved in the condensations, such as amino, carboxy or hydroxyl, may be protected by methods standard in peptide chemistry prior to the coupling reactions and subsequently deprotected to obtain the desired products.

Reactive group protection which can be employed during coupling reactions encompass known techniques such as, for example, by N-formyl, N-t-butoxycarbonyl, N-carbobenzyloxy, and O-benzyl groups followed by their removal to yield (I). Furthermore, the R and $R_3$ functions may include removable ester groups such as benzyl, ethyl, or t-butyl.

The starting materials required for the processes of the invention are known in the literature or can be made by known methods from known starting materials.

The above described syntheses can utilize racemates or enantiometers as starting materials. When diastereomeric products result from the synthetic procedures, the diastereomeric products can be separated by chromatographic or fractional crystallization methods. The resolution of intermediates or end products if desired can be accomplished by the crystallization of salts formed from optically active acids or bases.

Many of the compounds of this invention form salts with various inorganic and organic bases which are also within the scope of the invention. Such cationic salts include alkali metal salts like sodium and potassium salts, alkaline earth metal salts like the calcium and magnesium salts, salts with organic bases e.g., dicyclohexylamine salts, N-methyl-D-glucamine, salts with amino acids like arginine and lysine. Those products which contain a basic function in $R_1$ or $R_2$ also afford salts with organic and inorganic acids such as maleic acid and hydrochloric acid. the non-toxic physiologically acceptable salts are preferred, although other salts are also useful, e.g., in isolating or purifying the product.

The salts may be formed by conventional means, as by reacting the free acid or free base forms of the product with one or more equivalents of the appropriate base or acid in a solvent or medium in which the salt is insoluble, or in a solvent such as water which is then removed *in vacuo* or by freeze-drying or by exchanging the cations of an existing salt for another cation on a suitable ion exchange resin.

The compounds of this invention inhibit angiotensin converting enzyme and thus block conversion of the decapeptide angiotensin I to angiotensin II. Angiotensin II is a potent pressor substance. Thus blood-pressure lowering can result from inhibition of its biosynthesis especially in animals and humans whose hypertension is angiotensin II related. Furthermore, converting enzyme degrades the vasodepressor substance, bradykinin. Therefore, inhibitors of angiotensin converting enzyme may lower blood-pressure also by potentiation of bradykinin. Although the relative importance of these and other possible mechanisms remains to be established, inhibitors of angiotensin converting enzyme are effective antihypertensive agents in a variety of animal models and are useful clinically, for example, in many human patients with renovascular, malignant and essential hypertension. See, for example, D. W. Cushman et al., *Biochemistry 16,* 5484 (1977).

The evaluation of converting enzyme inhibitors is guided by *in vitro* enzyme inhibition assays. For example, a useful method is that of Y. Piquilloud, A. Reinharz and M. Roth, *Biochem. Biophys. Acta, 206,* 136 (1970) in which the hydrolysis of carbobenzyloxyphenylalanylhistidinylleucine is measured. *In vivo* evaluations may be made, for example, in normotensive rats challenged with angiotensin I by the technique of J. R. Weeks and J. A. Jones, *Proc. Soc. Exp. Biol. Med., 104, 646* (1960) or in a high renin rat model such as that of S. Koletsky et al., *Proc. Soc. Exp. Biol. Med., 125,* 96 (1967).

Thus, the compounds of this invention are useful as antihypertensives in treating hypertensive mammals, including humans, and they can be utilized to achieve the reduction of blood pressure by formulating in compositions such as tablets, capsules or elixirs for oral administration or in sterile solutions or suspensions for parenteral administration. The compounds of this invention can be administered to patients (animals and human) in need of such treatment in dosages that will at least provide pharmaceutical effectiveness. Although the dose will vary depending on severity of disease, weight of patient and other factors which a person skilled in the art will recognize, the dosage range will generally be about 0.5 to 50 mg per kilo per day.

This dose range can be adjusted on a unit basis as necessary to permit divided daily dosage. Naturally, the dose will vary depending on the severity of the disease, concurrent medication and other factors which a person skilled in the art will recognize.

Also, the compounds of this invention may be given in combination with diuretics or other antihypertensives. Typically these are combinations whose individual per day dosages range from one fifth of the minimally recommended clinical dosages to the maximum recommended levels for the entities when they are given singly. To illustrate these combinations, one of the antihypertensives of this invention effective clinically in the range 5—500 milligrams per day can be effectively combined at levels ranging from 1—500 milligrams per day with the following antihypertensives and diuretics in dose ranges per day as indicated:

hydrochlorothiazide (10—200 mg), timolol (5—60 mg), methyl dopa (65—2000 mg), the pivaloyloxyethyl ester of methyl dopa (30—1000 mg), indacrinone and variable ratios of its enantiomers (25—150 mg) and (—)-4-{3-{-[2-( -hydroxycyclohexyl)-ethyl]-4-oxo-2-thiazolidinyl}-propyl}-benzoic acid (10—100 mg).

In addition, the triple drug combinations of hydrochlorthiazide (15—200 mg) plus amiloride (5—20 mg) plus converting enzyme inhibitor of this invention (1—500 mg) or hydrochlorothiazide (15—200 mg) plus timolol (5—50 mg) plus the converting enzyme inhibitor of this invention (1—5000 mg) are effective combinations to control blood pressure in hypertensive patients.

The above dose ranges will be adjusted on a unit basis as necessary to permit divided daily dosage. Also, the dose will vary depending on the severity of the disease, weight of patient and other factors which a person skilled in the art will recognize.

Typically, the compounds of this invention can be formulated into pharmaceutical compositions as described below.

About 5 to 500 mg. of a compound or compounds of Formula I or a physiologically acceptable salt is compounded with a physiologically acceptable vehicle, carrier, excipient, binder, preservative, stabilizer, flavor, etc., in a unit dosage form as called for by accepted pharmaceutical practice. The amount of active substance in these compositions or preparations is such that a suitable dosage in the range indicated is obtained.

Illustrative of the adjuvants which may be incorporated in tablets, capsules and the like are the following: a binder such as gum tragacanth, acacia, corn starch, or gelatin; an excipient such as microcrystalline cellulose; a disintegrating agent such as corn starch, pregelatinized starch, alginic acid and the like; a lubricant such as magnesium stearate; a sweetening agent such as sucrose, lactose or saccharin; a flavoring agent such as peppermint, oil of wintergreen or cherry. When the dosage unit form is a capsule, it may contain in addition to materials of the above type, a liquid carrier such as fatty oil. Various other materials may be present as coatings or to otherwise modify the physical form of the dosage unit. For instance, tablets may be coated with shellac, sugar or both. A syrup or elixir may contain the active compound, sucrose as a sweetening agent, methyl and propyl parabens as preservatives, a dye and a flavouring such as cherry or orange flavor.

Sterile compositions for injection can be formulated according to conventional pharmaceutical practice by dissolving or suspending the active substance in a vehicle such as water for injection, a naturally occurring vegetable oil like sesame oil, coconut oil, peanut oil, cottonseed oil, etc. or a synthetic fatty vehicle like ethyl oleate or the like. Buffers, preservatives, antioxidants and the like can be incorporated as required.

The following examples are illustrative of the invention and constitute especially preferred embodiments. The preferred diastereomers of these examples, which are at both carbon atoms bearing $R_1$ and $CO_2R_3$ of the natural L-amino acid configuration, are isolated by column chromatography or fractional crystallization. In most cases, these preferred absolute configurations can also be designated (S)—.

In the following Examples, NMR chemical shifts are reported in ppm downfield from tetramethyl silane as internal standard. Optical rotations were measured in methanol solution.

## Example 1

α-Azaalanyl-(L)-proline t butyl ester (*2*)

A solution of proline t-butyl ester (10.0 g; 0.058 mol) and triethylamine (8.56 ml; 6.22 g; 0.0614 mol) in dry THF (60 ml) was added to a cold (0°C) solution of phosgene (12.5%) in benzene (61 ml; 0.070 mol) over 1 hour. An additional 50 ml of dry THF was added and stirring was continued for 2 hours. The reaction mixture was filtered and evaporated, giving N-chlorocarbonyl-(L)-proline t-butyl ester (1) as a dark-red oil (13.35g).

A solution of crude carbamyl chloride *1* (9.35g) in dry THF (40 ml) was added to an ice-cold solution of methylhydrazine (3.46 g; 4.00 ml; 0.075 mol) and triethylamine (6.33g; 8.73 ml; 0.063 mol) in dry THF (40 ml). Stirring was continued at room temperature for 18 hours. The reaction mixture was filtered and evaporated, giving a light yellow solid. Recrystallization from hexane — chloroform gave *2* (6.62 g; 0.0272 mol; 68%) as a white solid, mp 89—92°, TLC (silica gel; $CH_2Cl_2$—$CH_3OH$, 10:1) Rf=0.45. MS m/e 243 ($M^+$). Analysis: Calculated for $C_{11}H_{21}N_3O_3$; C, 54.30; H, 8.70; N, 17.27. Found: C, 54.45; H, 8.96; N, 17.29, NMR ($CDCl_3$): δ 1.42 (9H,S); 1.7—2.3 (4H,S); 3.00 (3H, S); 3.4—3.8 (2H, m); 3.8 (2H, bs); 4.4 (1H.m) 1R ($CHCl_3$): 3500—3300, 1730, 1630 $cm^{-1}$ $[\alpha]_D$ −55.7°±0.5°.

## Example 2

α-Azaglycinyl-(L)-proline t-butyl ester (*3*)

A solution of carbamoyl chloride *1* (1.00 g; from above) in dry THF (3.5 ml) was added over 10 minutes to an ice-cold solution of anhydrous hydrazine (0.269 g; 0.310 ml; 8.40 mmol) and triethylamine (0.708 g; 0.975 ml; 7.00 mmol) in dry THF (4 ml). Stirring was continued overnight at room temperature. the mixture was filtered and evaporated to a light yellow solid. Recrystallization from hexanes-ethyl/acetate gave pure carbazide *3* (0.533 g; 2.33 mmol; 52%) as a white solid, mp 83—84°C. TLC (silica gel; $CH_2Cl_2$—$CH_3OH$, 10:1)Rf=0.25 0.35. MS m/e 173 ($M^+$—56). Analysis: Calculated for $C_{10}H_{19}N_3O_3$: C, 52.39; H, 8.35; N, 18.33. Found: C, 52.08; H, 8.58; N, 18.01; NMR ($CDCl_3$); 1.43 (9H.S); 2.0—2.3 (4H, m); 3.1—3.4 (2H, m); 3.8(2H, bs): 4.3—4.6(1H, m); 6.2(1H, bs); IR($CHCl_3$): 3500—3300, 1730, 1660, 1640 $cm^{-1}$. $[\alpha]_D$ −46.5±0.5°.

## Example 3

### N-Carbobenzyloxy-N'-isopropylhydrazine (*4*)

Acetone (0.377g; 0.477 ml; 6.50 mmol) was added to a solution of carbobenzyloxyhydrazine (1.00 g; 6.02 mmol) in absolute ethanol (6 ml). After one hour, the solution was concentrated to dryness and then ethanol (5 ml) and sodium cyanoborohydride (0.19 g; 3.0 mmol) were added. The mixture was stirred for 18 hours and then evaporated to a thick syrup. Hydrochloric acid (0.50 N; 5 ml) and ether (50 ml) were added and the two-phase mixture was stirred for one hour. The aqueous layer was neutralized by addition of solid $NaHCO_3$ and extracted with ether (2 × 25 ml). The combined ether portions were washed with water and brine and dried ($MgSO_4$). Removal of solvent gave a solid which was recrystallized from hexanes — ether, giving pure hydrazide *4* (1.09g; 5.25 mmol; 87%), mp 61—62°. TLC (silica gel; $CH_2Cl_2$—$CH_3OH$; 20:1) Rf=0.5. MS m/e 208 (M+). Analysis: Calculated for $C_{11}H_{16}N_2O_2$: (C, 63.44; H, 7.44; N, 13.45). Found: C, 63.22; H, 7.67; N, 13.71; NMR ($CDCl_3$); 0.97 (6H, d, J=6); 3.15 (1H, septet, J=6); 3.8—4.6 (1H, broad); 5.13 (2H,S); 6.2—7.0 (1H, broad); 7.33 (5H,S); IR($CHCl_3$): 3500, 1720 $cm^{-1}$.

## Example 4

### α-Azavalinyl-(L)-proline t-butyl ester (*5*)

Carbamoyl chloride *1* (4 mmol, crude), prepared as described above, was combined with carbazide *4* (3.50 mmol; 0.728g) and triethylamine (0.557 ml; 4.00 mmol) in dry THF (2 ml). The mixture was heated at reflux for 7 hours, then filtered and evaporated to a thick oil. This was dissolved in benzene (10 ml) and hydrogenated with 10% Pd (C) (0.35g) at 1 atm. for 6 hours. Filtration of the catalyst and evaporation gave a light yellow oil which was purified by flash chromatography on silica gel ($CH_2Cl_2$—$CH_3OH$, 50:1). There was obtained pure carbazide *5* (0.622g; 2.30 mmol; (66%) as a light yellow solid. Recrystallization from hexanes gave a sample, mp 87—91°C. TLC (silica gel; $CH_2Cl_2$—$CH_3OH$; 20.1) Rf=0.4. MS m/e 271 ($M^+$). Analysis: Calculated for $C_{13}H_{25}N_3O_3$: C, 57.54; H, 9.29; N, 15.48. Found: C, 57.67; H, 9.46; N, 15.44. NMR ($CDCl_3$): 1.08 (3H, d, J=6); 1.13 (3H, d, J=6); 1.43 (9H, s); 1.7—2.2 (4H, m); 2.4—2.7 (2H, m); 3.3 (2H, bs); 4.07 (1H, septet, J=6); 4.3—4.5 (1H, m). 1R ($CHCl_3$): 3600—3300, 1730, 1630 $cm^{-1}$. $[\alpha]_D$ = 15.9° ±0.5°.

## Example 5

### N-t-BOC-2-pyrrolidinol (*6*)

Di-*t*-butyl dicarbonate (2.3 ml; 10 mmol) was added dropwise to a solution of 4-aminobutyraldehyde diethyl acetal (1.73 ml; 10 mmol) in chloroform (20 ml). Warming of the solution and evolution of a gas were observed. After 10 minutes, TLC (silica gel, $CH_2Cl_2$—$CH_3OH$, 20:1) showed complete consumption of the starting amine and a single product spot, Rf=8. Solvent was removed, giving a colorless oil.

The crude product was dissolved in a mixture of THF (30 ml) and hydrochloric acid (0.50 N, 30 ml). Stirring was continued for 2 hours. Then the mixture was diluted with brine (50 ml) and extracted with ether (3 × 25 ml). The combined ether portions were washed with water, saturated bicarbonate, and brine, and dried ($MgSO_4$). After removal of solvent, the colorless oil was purified by chormatography on silica gel (Baker; 2 × 18 cm) in hexanes — ether (2:1), giving pure *6* (0.35g, 1.87 mmol, 19%) as a colorless oil. TLC (silica gel, hexanes-ether, 2:1) Rf=0.5, MS m/e 170 ($m^+$—OH). NMR ($CDCl_3$): 1.48 (9H, S); 1.8—2.1 (4H, m); 3.2—3.7 (2H, m); 4.2—4.4 (1H, broad); 5.4—5.6 (1H, broad). IR ($CHCl_3$): 3700—3400, 3000, 2920, 1675 $cm^{-1}$.

## Example 6

### N-(t-Boc-4-amino-1-butyl-N'-CBZ-hydrazine (*8*)

A solution of N-t-Boc-2-pyrrolidinol (0.187 g; 1.00 mmol) and carbobenzyloxyhydrazine (0.166 g; 1.00 mmol) were combined with dry THF (2 ml) and the resulting solution was heated at reflux for 2 hours. TLC (silica gel; hexanes — EtAc, 2:1) showed two carbazone isomers *7a, 7b*, Rf=0.2, 0.3. The mixture was evaporated to dryness and the residue was combined with ethanol (3 ml) and sodium cyanoborohydride (63 mg; 1.00 mmol). the solution was stirred for 3 hours and then the solvent was evaporated and replaced with hydrochloric acid (0.50N, 3 ml) and ether (15 ml). the two-phase mixture was stirred for 30 min. and then the aqueous layer was neutralized with solid $NaHCO_3$ and extracted with ethyl acetate (3 × 10 ml). The combined organic portions were dried ($MgSO_4$) and solvent was removed, giving a light yellow oil. Chroatography on silica gel (Baker, 1 × 20 cm) in hexanes — EtAc (2:1) gave pure carbazide *8* (0.155 g; 0.46 mmol; 46%) which was recrystallized from hexanes—EtAc, mp 73—74°. TLC (silica gel; hexanes — EtOAc; 2:1) Rf=0.25. MS m/e 281 ($M^+$—56). Analysis: Calculated for $C_{17}H_{27}N_3O_4$: C, 60.51, H, 8.06; N, 12.45. Found: C, 60.18; H, 8.11; N, 12.26 NMR ($CDCl_3$): 1.42 (9H, s); 2.8—3.3 (4H, m); 3.9—4.1 (1H, broad); 4.8—5.0 (1H, broad); 5.17 (2H, s); 6.7—6.9 (1H, broad); 7.37 (5H, s); 1R ($CHCl_3$) 3500, 2980, 1720 $cm^{-1}$.

## Example 7

### N-t-Boc-α-Azalysyl-(L)-proline t-butyl ester (*10*)

A solution of carbazide *8* (0.941 g; 2.79 mmol) and carbamoyl chloride *1* (4 mmol.; crude) in dry THF (7 ml) containing triethylamine (0.56 ml; 4.00 mmol) was warmed at reflux for 8 hours. TLC (silica gel, hexanes-EtAc, 1:1, 2 developments) showed a single product (*9*), Rf=0.5. The residue after filtration and evaporation of solvent was dissolved in a mixture of ethyl acetate (10 ml) and acetic acid (3 ml) and hydrogenated at 1 atm with 10% Pd(C) (0.20 g) for 4 hours. The crude product was purified by flash chromatography on silica gel (E. Merck No. 9385, 1.5 × 22 cm) in hexanes — EtOAc (2:1), giving pure

carbazide *10* (0.579 g; 1.45 mmol; 52%) as a colorless oil. TLC (silica gel, EtOAc) Rf=0.3. MS m/e 400 ($M^+$). NMR ($CDCL_3$): 1.43 (18H, s); 1.8—2.1 (8H, m); 3.0—3.8 (8H, m); 4.3—4.7 (1H, m); 4.8—5.1 (1H, broad). IR ($CHCl_3$): 3600—3300, 1730 (shoulder), 1720, 1630 $cm^{-1}$.

Example 8

α-Azaalanyl-(L)-phenylalanine t-butyl ester (*11*)

Triethylamine (0.28 ml; 2 mmol) was added to a slurry of (L)-phenylalanine t-butyl ester hydrochloride (0.50 g; 1.94 mmol) in methylene chloride (4 ml). Carbonyldiimidazole (0.325 g; 2 mmol) was added, and stirring was continued for 30 minutes. Then methylhydrazine (0.11 ml; 2 mmol) was added over 5 minutes. Stirring was continued for 18 hours. Solvent was removed, and the resulting solid residue was partitioned between ether and water. The ether layer was washed twice with water and once with brine, and dried ($MgSO_4$). Removal of solvent gave crude carbazide *11* as a colorless oil. Flash chromatography on silica gel (E. Merck No. 9385; 2 × 12 cm) in 100:1 methylene chloride: methanol gave pure *11* (0.480 g; 1.63 mmol); 84%): TLC (20.1 $CH_2Cl_2$:$CH_3OH$) Rf=0.3. MS m/e 294 (M+). Analysis: Calculated for $C_{15}H_{24}N_3O_3$: C, 61.20 H, 8.22 N, 14.27. found: C, 61.75 H, 8.28 N, 14.00. NMR ($CDCl_3$) 1.33 (9H, s); 3.00 (3H, s); 3.05 (2H, d, J=6); 3.6 (2H, bs); 4.63 (1H, d of t; J=8,6); 6.7—7.0 (1H, m); 7.25 (5H, s); IR ($CHCl_3$): 3450, 3000, 1730, 1650 $cm^{-1}$.

Example 9

α-Azaphenylalanyl-([L)-proline t-butyl ester (*12*)

Carbamoyl chloride *1* was prepared as before from (L)-proline t-butyl ester (10 g), giving a light-red oil (15.7 g). This was added as a solution in THF (50 ml) to an ice-cold mixture consisting of benzylhydrazine dihydrochloride (0.029 mol; 5.7 g), triethylamine (0.088 mol; 12.2 ml) and THF (40 ml). The mixture was stirred at room temperature for 18 hours. Filtration and removal of solvent gave the crude product as a thick syrup. This was purified by flash chromatography on silica gel (E. Merck No. 9385; 3 × 30 cm) in $CH_2Cl_38$—$CH_3OH$ (100:1). Fractions containing pure carbazide *12* were combined, giving 4.06 g (.0127 mol; 44%). Early fractions contained a small amount of an isomeric carbazide *13*, but this was not fully characterized. TLC (20:1 $CH_2Cl_2$:$CH_3OH$) Rf=0.5. MS m/e 319 ($M^+$). NMR ($CDCl_3$) 1.42 (9H, s); 1.8—2.2 (4H, m); 3.4—3.5 (2H, bs); 3.5—3.8 (2H, m); 4.3—4.6 (1H, m); 4.62 (2H, s); 7.3—7.4 (5H, s). IR ($CHCl_3$): 3000, 1740, 1630 $cm^{-1}$.

Example 10

N-(1-Carbobenzyloxy-3-phenylpropyl)-α-azaalanyl-(L)-proline t-butyl esters — (*14R, 14S*)

Carbazide *2* (0.243 g; 1.00 mmol) and benzyl 4-phenyl-2-oxobutanoate (0.268 g; 1.00 mmol) were combined with THF (2 ml) and 2 drops of glacial acetic acid and the resulting solution was warmed at 60° for one hour. After the mixture had been stirred overnight at room temperature, it was evaporated to a thick, yellow syrup. TLC (silica gel; $CH_2Cl_2$—$CH_3OH$ 20:1) showed a single product spot at Rf=0.6. To a solution of the crude product in absolute ethanol (3 ml) was added sodium cyanoborohydride (63 mg; 1.00 mmol). After 3 hours the ethanol was evaporated and the residue was combined with dilute hydrochloric acid (0.5 N, 3 ml) and ethyl acetate (15 ml). The two-phase mixture was stirred vigorously for one hour and then the aqueous phase was neutralized by addition of solid potassium carbonate and extracted three times with ethyl acetate. The combined organic portions were washed with water and brine and dried ($MgSO_4$). Removal of solvent gave a thick, colorless oil. TLC (silica gel; hexanes-ethyl acetate 2:1; 2 developments) showed two diastereomers, Rf=0.25, 0.30. Separation of the two was achieved by chromatography on silica gel (E. Merck 60B; 6.5 ml/min) at medium pressure in hexanes-ethyl acetate (4:1).

The following were obtained:

1) *14S*; more-mobile diastereomer (158 mg). MS m/e 495 ($M^+$). NMR ($CDCl_3$) 1.42 (9H, s); 1.8—2.3 (6H, m); 2.5—2.9 (2H, m); 2.90 (3H, s); 3.1—3.6 (3H, m); 4.3—4.6 (1H, m); 5.0 (1H, broad); 5.10 (2H, s); 7.10 (5H, s); 7.28 (5H, s). IR ($CHCl_3$): 3000, 1740, 1640 $cm^{-1}$.

2) Mixed fractions 53.5 mg.

3) *14R*; less mobile diastereomer (170 mg) mp 76°—79° (hexanes-EtAc) MS m/e 495 ($M^+$). NMR ($CDCl_3$) 1.42 (9H, s); 1.7—2.2 (6H, m); 2.5—2.9 (2H, m); 2.92 (3H, s); 3.1—3.6 (3H, m); 4.2—4.5 (1H, m); 5.07 (2H, s); 5.1—5.3 (1H, broad); 7.10 (5H, s); 7.27 (5H, s). IR ($CHCl_3$): 3000, 1740, 1640 $cm^{-1}$. (A total of 382 mg; 0.771 mmol; 77%). Analysis: Calculated for $C_{28}H_{37}N_3O_5$: C, 67.86; H, 7.52; N, 8.48. Found: C, 67.74; H, 7.39; N, 8.29.

Example 11

N-(1-Carbobenzyloxy-3-phenylpropyl)-α-azaalanyl-(L)-prolines (*15R, 15S*)

A solution of the more mobile azapeptide ester *14S* (158 mg) in trifluoroacetic acid (10 ml) was allowed to stand for 3 hours. The solution was evaporated to dryness, and then an ether solution of the residue was washed several times with water and dried ($MgSO_4$). Removal of solvent gave a thick residue which was purified on a column of LH=20 (in methanol) to give 118 mg of azapeptide *15S* as a colorless oil. TLC (silica gel; ethyl acetate-pyridine-acetic acid-water (EPAW) 20:5:1:1) Rf=0.6.

NMR ($CDCl_3$) 1.7—2.3 (6H, m); 2.5—2.9 (2H, m); 2.95 (3H, s); 3.3—4.2 (3H, m); 4.4—4.8 1H, m); 5.3 (2H, s); 7.1—7.4 (5H, m); 7.30 (5H, s).

The less mobile azapeptide diastereomer *14R* (170 mg) was treated in an identical manner, and the

resulting product was purified by chromatography on LH=20 (in methanol), giving 92 mg of pure azapeptide *15R*. TLC (silica gel; EPAW 20:5:1:1) Rf=0.6.

NMR ($CDCL_3$) 1.6—2.2 (6H, m); 2.5—2.9 (2H.m); 3.08 (3H, s); 3.3—3.6 (2H, m); 3.8—4.1 (1H, m); 4.7—4.9 (1H, m); 5.02 (2H, s); 5.8—6.0 (1H, broad); 7.1—7.3 (5H, m); 7.18 (5H, s).

Example 12

N-(1-Carboethoxy-3-phenylpropyl)-α-azaalanyl-(L)-proline t-butyl esters *(16R, 16S)*

Using the procedure described in Example 11 above, carbazide *2* (0.486 g; 2.00 mmol) and ethyl 4-phenyl-2-oxobutanoate (0.412 g; 2.00 mmol) were used to prepare azapeptide *15* (R, S), which was purified by flash chromatography on silica gel (E. Merck, No. 9385; 2 × 25 cm) in $CH_2Cl_2$—$CH_3OH$ (100:1). The product (0.753 g, 1.74 mmol; 87%) was a colorless oil. Separation of diastereomers was achieved on a column (3 × 18 cm) of E. Merck silica gel H (Acc. to Stahl), slurry-packed in hexanes — EtAc (3:1) by pipette. More mobile isomer (116 mg), less-mobile isomer (75.4 mg) and mixtures of the two (300 mg) were obtained. More mobile isomer *16S*: TLC (silica gel, hexanes — EtAc, 2:1, 2 developments, Rf=0.30. MS m/e 433 ($M^+$). NMR ($CDCl_3$): 1.25 (3H, t, J=7); 1.43 (9H, s); 1.7—2.3 (6H, m); 2.6—3.0 (2H, m); 2.95 (3H, s); 3.4—3.7 (3H, m); 4.12 (2H, q, J=7); 4.45 (1H, broad q, J=6); 4.8—5.0 (1H, broad); 7.2—7.4 (5H, bs); IR ($CHCl_3$): 1730, 1640 $cm^{-1}$. Less mobile isomer (16R): TLC, Rf=0.25. MS: m/e 433 ($M^+$), NMR ($CDCl_3$): 1.23 (3H, t, J=7); 1.42 (9H, s); 1.8—2.4 (6H, m); 2.6—3.0 (2H, m); 2.95 (3H, s); 3.4—3.7 (3H, m); 4.12 (2H, q, J=7); 4.3—4.5 (1H, m); 5.0—5.3 (1M, broad); 7.2—7.4 (5H, bs). IR ($CHCl_3$): 1730, 1640 $cm^{-1}$.

Example 13

N-(1-Carboethoxy-3-phenylpropyl)-α-azaalanyl-(L)-prolines *(17R, 17S)*

Each diastereomer of azapeptide was treated as described in Example 11 above with trifluoroacetic acid with the following results. Azapeptide *16S* (0.116 g; 0.238 mmol) gave azapeptide *17S* (0.0448 g; 0.119 mmol; 50%) after purification on LH-20 (in methanol). TLC (silica gel, EPAW, 20:5:1:1) Rf=0.65. MS m/e 449 ($M^+$-monotrimethylsilyl) NMR ($CDCl_3$): 1.28 (3H, t, J=7); 1.8—2.4 (6H., m); 2.5—2.9 (2H, m); 3.07 (3H, s); 3.5—4.0 (3H, m); 4.17 (2H, q, J=7); 4.3—4.7 (1H, m); 7.23 (5H, bs); IR ($CHCl_3$): 3500—2900; 1740; 1670 $cm^{-1}$. Azapeptide *16R* (0.0754 g; 0.174 mmol) gave azapeptide *17R* (.0398; 0.106 mmol; 61%) after purification on LH-20. TLC, RF=0.65. MS m/e 449 ($M^+$-monotrimethylsilyl). NMR ($CDCl_3$: 1.28 (3H, t, J=7); 1.8—2.4 (6H, m); 2.7—3.0 (2H, m); 3.10 (3H, s); 3.5—4.0 (3H, m); 4.17 (2H, q, J=7); 4.5—4.9 (1H, m); 7.15 (5H, m), IR ($CHCl_3$): 3500—2900; 1740, 1670 $cm^{-1}$.

Example 14

N-(1-Carboxy-3-phenylpropyl)-α-azaalanyl-(L)-proline t-butyl ester *(18)*

A solution of carbazide *2* (0.243 g; 1.00 mmol) and 4-phenyl-2-oxobutanoic acid (0.534 g; 3.00 mmol) in water (1 ml) was adjusted to pH=7 by addition of aqueous sodium hydroxide (2N). Then a solution of sodiumcyanoborohydride (.0628 g; 1.00 mol) in water (1 ml) was added over a period of 4 hours. Stirring was continued for 48 hours. Water-washed DOWEX$^R$ 50W—X4 resin (3 g) was added and stirring was continued for 90 minutes. The mixture was then diluted with water-$CH_3OH$ (1:1; 5 ml) and transferred to a column of the same resin (5 g). Elution was carried out with $H_2O$—$CH_3OH$ (10:1) and then $H_2O$ — pyridine (50:1). The latter solvent mixture brought about elution of pure azapeptide ester *18* (.300 g; 0.742 mmol; 74%) as a white foam after freeze-drying. TLC (silica gel; EPAW 20:5:1:1), Rf=0.6. MS m/e 477 ($M^+$, monotrimethylsilyl). NMR ($CDCl_3$); 1.43 (9H, S); 1.7—2.3 (6H, m); 2.6—3.0 (2H, m); 3.00 (3H, s); 3.4—3.7 (2H, m); 4.3—4.6 (1H, m); 7.20 (5H, s); 7.25 (2H, bs).

Example 15

N-(1-Carboxy-3-phenylpropyl)-α-azaalanyl-(L)-proline *(19)*

A solution of azapeptide ester *18* (0.301 g; .742 mmol) in EtAc (2 ml) was added by syring to an ice-cold solution of anhydrous chloride in EtAc (5 ml; 5.16N). Stirring was continued for 2 hours at room temperature, and then the solution was evaporated to a white solid. Purification was carried out on DOWEX$^R$ 50W—X4 resin (7 g) by eluting with $H_2O$—$CH_3OH$ (1:1, then 10:1) and then by $H_2O$-pyr (50:1). The latter solvent mixture brought about elution of azapeptide *19* (0.100 g; 0.287 mmol; 39%) as a white foam. Further purfication by chromatography on a column of LH-20 (in $CH_3OH$) gave pure azapeptide *19* (71 mg) as a white foam. TLC (silica gel; EPAW, 10:5:1:3) RF=0.5.

Example 16

N-(1-Carboxyethyl)-α-azaalanyl-(L)-proline-t-butyl ester *(20)*

A solution of carbazide *2* (0.486 g; 2.00 mmol) and sodium pyruvate (1.10 g; 10 mmol) in water (30 ml) was stirred for 15 minutes. A solution of sodium cyanoborohydride (0.251 g; 4.00 mmol) in $H_2O$ (2 ml) was added over 4 hours. Stirring was continued for 18 hours. Then 5 g of DOWEX$^R$ 50W—X4 resin ($H_2O$-washed) was added, and the mixture was stirred for 4 hours. The mixture was added to a column of the same resin (5 g) and eluted with $H_2O$—$CH_3OH$ (10:1) and then $H_2O$-pyr (50:1). The latter solvent caused elution of pure azapeptide *20* (0.504 g; 1.60 mmol; 80%) which was freeze dried to a white foam. TLC (silica gel; EPAW 20:5:1:1) Rf=0.7. NMR ($CDCl_3$) 1.30 (3H, d, J=7), 1.45 (9H, s); 1.8—2.3 (4H, m); 3.03 (3H, s); 3.5—3.9 (3H, m); 4.4—4.7 (1H, m); 8.2—8.5 (1H, broad).

Example 17

N-(1-Carboxyethyl)-α-azaalanyl-(L)-proline *(21)*

A solution of azapeptide *20* (0.504 g; 1.60 mmol) in ethyl acetate (2.5 mol) was added to an ice-cold solution of hydrogen chloride in ethyl acetate (7.5 ml; 5.2 N). The resulting solution was stirred at room temperature for 4 hours. Removal of solvent gave a white, solid residue. Purification of this solid on DOWEX$^R$ 50W—X4 (7.5 g) with $H_2O$-pyr (50:1) as eluant gave, after freeze drying, pure azapeptide *21* (0.290 g; 1.12 mmol; 70%) as a white foam. Purification of a portion of this material (50 mg) on a column of LH-20 (in methanol) gave azapeptide *21* as a colorless oil (20.0 mg). TLC (silica gel, EPAW, 10:5:1:3) Rf=0.25, 0.30 (2 diastereomers).

Example 18

N-(1-Carboxy-3-methylbutyl)-α-azaalanyl-(L)-proline-t-butyl ester *(22)*

To a solution of carbazide *2* (0.468 g; 2.00 mmol) and 4-methyl-2-oxopentanoic acid (1.52 g, 10.0 mmol) in $H_2O$ (4 ml) was added over 7 hours a solution of sodium cyanoborohydride (0.251 g; 4.00 mmol) in $H_2O$ (2 ml). Stirring was continued for 18 hours. Water-washed DOWEX$^R$ 50W—X4 resin (7.5 g) was added to the mixture (pH=2) and vigorous stirring was continued for 2 hours. Then the entire mixture was transferred to a column of DOWEX$^R$ resin (5 g) using $H_2O$—$CH_3OH$ (1:1) to dissolve some insoluble material remaining in the flask. The column was eluted with $H_2O$—$CH_3OH$ (1:1, then 10:1) and then $H_2O$-pyr (50:1). This last solvent caused elution of pure azapeptide *22* (0.596 g; 1.67 mmol) 84%) which was a white foam after freeze-drying. TLC (silica gel; EPAW, 20:5:1:1) Rf=0.80, 0.85 (2 diastereomers). MS m/e 429 ($M^+$, monotrimethylsilyl). NMR ($CDCl_3$): 0.95 (6H, d, J=6); 1.38 (9H, s); 1.8—2.2 (7H, m); 3.03 (3H, s); 3.3—3.6 (3H, m); 4.2—4.5 (1H, m); 7.6—7.8 (2H, broad). IR ($CHCl_3$): 3600—2500, 1740, 1650 $cm^{-1}$.

Example 19

N-(1-Carboxybutyl)-α-azaalanyl-(L)-proline *(23)*

A solution of azapeptide *22* (74.4 mg; 0.208 mmol) in trifluoroacetic acid (2 ml) was allowed to stand for 2 hours. Then solvent was evaporated and the residue was purified on DOWEX$^R$ 50W—X4 resin (3 g). Elution with $H_2O$-pyr (50:1) gave azapeptide *23* (56.9 mg) 0.189 mmol, 91%); as a white foam. Further purification of a column LH-20 (in $CH_3OH$) gave 28.3 mg of a white foam. TLC (silica gel; EPAW, 10:5:1:3) Rf=0.5, 0.6 (2 diastereomers). MS m/e 445 ($M^+$, bistrimethylsilyl): NMR ($CDCl_3$): .98 (6H, d, J=6); 1.4—1.8 (2H, m); 1.8—2.3 (5H, m); 3.10 (3H, s); 3.4—3.8 (3H, m); 4.3—4.7 (1H, m); 9.0—9.7 (3H, broad). IR ($CHCl_3$): 3600—2500; 1720, 1670, 1610 $cm^{-1}$.

Example 20

N-(1-Carbobenzyloxy-5-phthalimidopentyl)-α-azaalanyl-(L)-proline-t-butyl ester *(24)*

A solution of carbazide *2* (0.665 g; 2.74 mmol) and benzyl 6-phthalimido-2-oxohexanoate (1.00 g; 2.74 mmol) in THF (5 ml) containing glacial acetic acid (3 drops) was heated at reflux for 3 hours. TLC (silica gel, $CH_2Cl_2$—$CH_3OH$, 20:1) showed a single product spot, Rf=0.5. The crude product was treated as for *14* (Example 10) with sodium cyanoaborohydride (172 mg; 2.74 mmol), giving pure carbazide *24* (1.18 g; 2.00 mmol; 73%) after flash chromatography on silica gel (hexanes — EtAc, 1:1). TLC (silica gel; hexanes — EtOAc, 1:1) showed a mixture (1:1) of diastereomers, Rf=0.25, 0.30. MS m/e 592 ($M^+$). Analysis: Calculated for $C_{32}H_{40}N_4O_7$: C, 64.85; H, 6.80; N, 9.45. Found: C, 64.24; H, 6.98; N, 8.95. NMR ($CDCl_3$): 1.40 (9H, s); 1.5—2.0 (8H, m); 2.88, 2.90 (3H, 2 × S); 3.2—3.7 (6H, m); 4.2—4.4 (1H, m); 4.8—5.1 (1H, m); 5.12, 5.15 (2H, 2 × S); 7.27 (5H, s); 7.6—7.9 (4H, m). IR ($CHCl_3$): 2980, 1770, 1730, 1720, 1640 $cm^{-1}$.

Example 21

N-(1-Carboxy-5-phthalimidopentyl)-α-azaalanyl-(L)-proline-t-butyl ester *(25)*

A solution of azapeptide ester *24* (0.402 g; 0.679 mmol) in ethyl acetate (5 ml) and acetic acid (0.2 ml) was hydrogenated with 110 mg of 10% Pd/C for 18 hours. Filtration of catalyst and removal of solvent gave azapeptide *25* as a white foam (0.330 g). TLC (silica gel; EPAW 20:5:1:1) Rf=0.7. MS m/e 574 ($M^+$, monotrimethylsilyl). NMR ($CDCl_3$): 1.42 (9H, s); 1.5—2.1 (10H, m); 3.00 (3H, s); 3.4—3.8 (5H, m); 4.3—4.6 (1H, m); 7.6—7.8 (4H, m); 7.8—8.0 (1H, broad).

Example 22

N-(5-Amino-1-carboxypentyl)-α-azaalanyl-(L)-proline *(27)*

To a solution of the crude azapeptide *25* (0.330 g) in ethanol (4 ml) was added anhydrous hydrazine (1.70 mmol; 0.054 g). After 3 hours, the mixture was filtered to remove the white precipitate and evaporated to a white foam. Purification on DOWEX$^R$ 50W—X4 (7.5 g) using $H_2O$-pyr (50:1) as eluant, gave N-(5-amino-1-carboxypentyl)-α-azaalanyl-(L)-proline t butyl ester *(26)* as a white foam (0.264 g). TLC (silica gel; EPAW 10:5:1:3) Rf=0.25, MS m/e 516 ($M^+$, bistrimethylsilyl).

A solution of the azapeptide ester *26* (0.264 g) in trifluoroacetic acid (5 ml) was allowed to stand for 2 hours. Solvent was removed, and the light-brown residue was chromatographed on DOWEX$^R$ 50W—X4 (10 g) using $H_2O$-pyr (50:1) as eluant. There was obtained 0.166 g of azapeptide *27* as a white foam. TLC (silica gel n-butanol, $H_2O$, acetic acid, EtOAc, 1:1:1:1) Rf=0.1—0.2. MS m/e 517 ($M^+$; tristrimethylsilyl — 15). NMR ($D_2O$); 1.2—2.2 (10, m); 2.85 (3H, s); 3.1—3.7 (5H, m); 4.0—4.4 (1H, m).

## Example 23

N-(1-Carboethoxy-3-phenylpropyl)-α-azaglycinyl-(L)-proline-t-butyl ester *(28)*

Using the procedure described in Example 10 above for *14*, carbazide *3* (0.458 g; 2.00 mmol) and ethyl 4-phenyl-2-butanoate (0.412 g; 2.00 mmol) were used to prepare azapeptide *28*, which was purified by flash chromatography on silica gel (0.633 g; 1.51 mmol; 76%). TLC (silica gel, $CH_2Cl_2$—$CH_3OH$, 10:1) Rf=0.75. MS m/e 419 ($M^+$) NMR ($COCl_3$): 1.22 (3H, t, J=7); 1.37 (9H, s); 1.8—2.3 (6H, m): 2.6—3.0 (2H, m); 3.2—3.8 (3H, m); 4.0—4.4 (1H, m); 4.08 (2H, q, J=7); 4.6—4.8 (1H, broad); 6.6 (1H, 6s); 7.17 (5H, 6s). IR ($CHCl_3$): 3500—3300, 1730, 1660 $cm^{-1}$.

## Example 24

N-(1-Carboethoxy-3-phenylpropyl)-α-azaglycinyl-(L)-proline *(29)*

Treatment of azapeptide *28* (0.284 g; 0.677 mmol) with trifluoroacetic acid (as described in Example 11; and purification on DOWEX$^R$ 50W—X4 (8 g) with $H_2O$-pyr (50:1) as eluant, gave after freeze-drying, pure azapeptide *29* (0.196 g; 0.541 mmol, 80%) as a white foam. TLC (silica gel, EPAW, 20:5:1:1) Rf=0.5. MS m/e 507 ($M^+$, disilyl). NMR ($CDCl_3$): 1.22 (3H, t, J=7); 1.7—2.4 (6H, m); 2.5—3.0 (2H, m); 3.1—3.9 (3H, m); 4.0—4.6 (1H, m); 4.08 (2H, q, J=7); 7.18 (5H, bs); 8.4—9.0 (2H, broad). IR ($CHCl_3$): 3600—2500; 1780 (shoulder); 1730, 1670, 1610 $cm^{-1}$.

## Example 25

N-(1-Carboxy-3-phenylpropyl)-α-azaglycinyl-(L)-proline *(30)*

To a solution of azapeptide *29* (0.132 g; 0.362 mmol) in ethanol (1.5 ml) was added in sodium hydroxide solution (0.80 ml; 0.80 mmol). The mixture was stirred for 24 hours and then placed onto a column of DOWEX$^R$ 50W—X4 (6 g). Elution with $H_2O$-pyr (50:1) gave, after freeze-drying, pure azapeptide *30* (0.080 g; 0.239 mmol; 66%) as a white powder. TLC (silica gel, EPAW, 10:5:1:3) Rf=0.45. MS m/e 551 ($M^+$, trisilyl). NMR ($CDCl_3$): 1.7—2.4 (6H, m); 2.7—3.1 (2H, m); 3.3—4.2 (4H, m); 7.1—7.3 (5H, bs); 8.4—9.4 (2H, broad). IR ($CHCl_3$): 3600—2500; 1780 (shoulder); 1730, 1670 $cm^1$.

## Example 26

Ethyl-phenyl-2-oxobutanoate (α-azaglycinyl)-(L)-proline t-butyl ester) carbazone *(31)*

A solution of carbazide *3* (0.458 g; 2.00 mmol) and ethyl 4-phenyl-2-oxobutanoate (0.412 g; 2.00 mmol) in THF (3 ml) was stirred overnight. The resulting carbazone was purified by flash chromatography on silica gel in $CH_2Cl_2$—$CH_3OH$ (100:1), giving 0.515 g (1.23 mmol; 62%) of *31* as a colorless oil. TLC (silica gel $_2I_2$—$CH_3OH$, 20:1) Rf=0.2—0.4. MS m/e 417 ($M^+$). IR ($CHCl_3$): 1740, 1720, 1660, 1610 $cm^{-1}$. Analysis: Calculated for $C_{22}H_{31}N_3O_5$: C, 63.29; H, 7.48; N, 10.06. Found: C, 63.69; H, 7.66; N, 10.02. NMR ($CDCl_3$): 1.25 (3H, t, J=7); 1.37 (9H, S); 1.7—2.1 (4H, m); 2.7—2.9 (4H, bs); 3.4—3.7 (2H, m); 4.13 (2H, q, J=7); 4.5—4.7 (1H, m); 7.2—7.4 (5H, bs).

## Example 27

Ethyl-4-phenyl-α-oxobutanoate (α-azaglycinyl-(L)-proline) carbazone *(32)*

A solution of carbazone *31* (80.0 mg; 0.192 mmol) in trifluoroacetic acid (3 ml) was allowed to stand for 2 hours. Removal of solvent gave a light-brown solid, which was recrystallized from hexanes-ethanol (2:1), giving pure carbazone *32* (31 mg; 0.085 mmol: 44%), mp 126—127°. TLC (silica gel, EPAW, 20:5:1:1) Rf=0.5. Analysis: Calculated for $C_{18}H_{23}N_3O_5$: C, 59.82; H, 6.41; N, 11.63. Found: C, 59.66; H, 6.44; N, 11.48. MS m/e 361 ($M^+$). NMR ($CDCl_3$): 1.32 (3H, t, J=7); 1.9—2.4 (4H, m); 2.80 (4H, bs); 3.4—3.8 (2H, m); 4.23 (2H, q, J=7); 4.5—4.8 (1H, m); 7.13 (5H, s). IR ($CHCl_3$): 3600—2500; 1760; 1740 (shoulder); 1670; 1630; 1580 $cm^{-1}$.

## Example 28

N-(1-Carboethoxy-3-phenylpropyl)-α-azaphenylalanyl-(L)-proline t-butyl ester *(34)*

A solution of carbazide *12* (0.568 g; 1.78 mmol) and ethyl 4-phenyl-2-oxobutanoate (0.367 g; 1.78 mmol) in absolute ethanol (2 ml) containing glacial acetic acid (3 drops) was heated at reflux for 4 hours. The resulting carbazone *33* was purified by flash chromatography on silica gel in $CH_2Cl_2$—$CH_3OH$ (100:1), giving pure material (0.387 g; 0.763 mmol); 43%) as a colorless oil. TLC (silica gel, $CH_2Cl_2$—$CH_3OH$, 20:1) Rf=0.7. MS m/e 507 ($M^+$). NMR ($CDCl_3$): 1.32 (3H, t, J=7); 1.40 (9H, s); 1.7—2.2 (4H, m); 2.68 (4H, s); 3.4—3.8 (2H, m); 4.22 (2H, q, J=7); 4.5—4.8 (1H, m); 4.93 (2H, s); 6.8—7.3 (5H, m); 7.13 (5H, s). IR ($CHCl_3$), 3000, 1730, 1660, 1600 $cm^{-1}$.

The carbazone *33* (0.387 g; 0.763 mmol) was treated for 24 hours with sodium cyanoborohydride (0.032 g; 0.509 mmol) in abosolute ethanol (3 ml). Workup as described in Example 10 gave azapeptide *34* as a colorless oil. Flash chromatography on silica gel in $CH_2Cl_2$—$CH_3OH$ (100:1) gave pure azapeptide (0.293 g, 0.575 mmol; 75%), TLC (silica gel, $CH_2Cl_2$—$CH_3OH$, 20:1) Rf=0.60, 0.65, (2 diastereomers, 1:1). IR ($CHCl_3$) 1740, 1630 $cm^{-1}$. MS m/e 509 ($M^+$). NMR ($CDCl_3$): 1.22 (3H, t, J=7); 1.42 (9H, s); 1.7—2.2 (6H, m); 2.4—2.8 (2H, m); 3.4—3.8 (2H, m); 4.03 (2H, q, J=7); 4.4—4.7 (3H, m); 7.22 (5H, bs); 7.32 (5H, bs).

## Example 29

N-(1-Carboethoxy-3-phenylpropyl)-α-azaphenylalanyl-(L)-proline *(35)*

A solution of azapeptide *34* (0.293 g; 0.575 mmol) in trifluoroacetic acid (6 ml) was allowed to stand for

2 hours. The solution was evaporated to dryness, and the light brown residue was dissolved in ether (25 ml). The solution was washed several times with brine, dried ($MgSO_4$) and evaporated, giving *35* as white foam (0.193 g; 0.427 mmol; 74%). TLC (silica gel; EPAW: 20:5:1:1) Rf=0.75. MS m/e 525 ($M^+$, monotrimethylsilyl). NMR ($CDCl_3$): 1.22 (3H, t, J=7); 1.7—2.3 (6H, m); 2.4—2.7 (2H, m); 3.4—3.8 (3H, m); 4.05 (2H, q, J=7); 4.1—4.4 (1H, m); 4.52 (2H, bs); 7.0—7.6 (10H, m). IR ($CHCl_3$): 3600—2500; 1740; 1680 $cm^{-1}$.

Example 30

N-(1-Carboxy-3-phenylpropyl)-α-azaphenylalanyl-(L)-proline *(36)*

To a solution of azapeptide *35* (0.140 g; 0.309 mmol) in absolute ethanol (1.5 ml) was added aqueous sodium hydroxide solution (0.70 ml; 0.70 mmol). Stirring was continued for 24 hours. The reaction mixture was placed onto a column of DOWEX$^R$ 50W—X4 (6 g). Elution with $H_2O$ and then $H_2O$-pyr (50:1) gave after freeze-drying, pure azapeptide *36* (0.0626 g; 0.147 mmol; 48%) as a white powder. TLC (silica gel; EPAW, 10:5:1:3) Rf=0.80, 0.85 (2 diastereomers). MS m/e 569 ($M^+$, bistrimethylsilyl). NMR ($CDCl_3$): 1.6—2.2 (6H, m); 2.4—2.8 (2H, m); 3.5—3.9 (3H, m); 4.3—4.7 (3H, m); 7.1—7.5 (10H, m). IR ($CHCl_3$): 3500—2900, 1730, 1650 $cm^{-1}$.

Example 31

N-(1-Carbobenzyloxy-3-phenylpropyl)-α-azavalinyl-(L)-proline t-butyl ester *(37)*

A solution of carbazide *36* (0.271 g; 1.00 mmol) and benzyl 4-phenyl-2-oxobutanoate (0.295 g; 1.10 mmol) in THF (3 ml) containing glacial acetic acid (2 drops) was warmed at reflux for 8 hours. TLC (silica gel; hexanes-EtAc, 2:1) showed product carbazones at Rf=0.50, 0.55. MS m/e 521 ($M^+$). The residue after evaporation was dissolved in ethanol and treated with sodium cyanoborohydride (63 mg; 1.00 mmol). After 4 hours, workup as described in Example 10 gave the product, which was purified by chromatography on silica gel (Baker 1.5 × 20 cm) in hexanes-EtOAc (10:1). TLC of the pure azapeptide (0.307 g; 0.587 mmol; 59%) on silica gel (hexanes — EtAc; 5:1) showed 2 diastereomers at Rf=0.30, 0.35 (1:1 mixture). MS m/e 523 ($M^+$). NMR ($CDCl_3$): 1.28, 1.30 (6H, pair of triplets, J=6); 1.42 (9H, s); 1.8—2.4 (6H, m); 2.6—3.0 (2H, m); 3.4—3.7 (4H, m); 4.3—4.7 (1H, m); 5.1—5.3 (2H, m); 7.17 (5H, bs); 7.33 (5H, s). IR ($CHCl_3$): 3000; 1740; 1650 $cm^{-1}$.

Example 32

1-(Carboxy-3-phenylpropropyl)-α-azavalinyl-(L)-proline *(39)*

A solution of azapeptide diester *37* (0.105 g; 0.201 mmol) in trifluoroacetic acid (4 ml) was allowed to stand for 2 hours. Solvent was evaporated, and a solution of the residue in ethyl acetate (25 ml) was washed several times with $H_2O$ and then dried ($MgSO_4$). The residue on evaporation (0.100 g) showed a single spot by TLC (silica gel; EPAW; 20:5:1:1) Rf=0.7. MS m/e 539 ($M^+$, monotrimethylsilyl). The crude azapeptide *(38)* was dissolved in ethyl acetate (3 ml) and glacial acetic acid (1 ml) and hydrogenated for 2 hours with 10% Pd/C (100 mg). Filtration of the catalyst and evaporation of solvent gave a white foam which was purified by chromatography on a column of LH-20 (in $CH_3OH$). The resulting azapeptide *39* (colorless oil) (52 mg; 0.138 mmol; 69%) showed a single spot on TLC (silica gel; EPAW, 20:1:1:1). MS m/e 521 ($M^+$, bistrimethylsilyl). NMR ($CDCl_3$): 1.0—1.4 (6H, m): 1.8—2.4 (6H, m); 2.6—3.0 (2H, m); 3.2—3.8 (4H, m): 4.2—4.6 (1H, m); 7.1—7.3 (5H, 6s). IR ($CHCl_3$): 3600—2500; 1730; 1640 $cm^{-1}$.

Example 33

$N^{\alpha}$-(1-Carbobenzyloxy-3-phenylpropyl)-$N^{\delta}$-t-Boc-α-azalysinyl-(L)-proline t-butyl ester *(41)*

Carbazide *10* (0.308 g; 0.779 mmol) and benzyl 4-phenyl-2-oxobutanoate (0.230 g; 0.866 mmol) were combined with dry tetrahydrofuran (2.5 ml) and the solution was heated at reflux for 4 hours. The product was filtered through silica gel (Baker, 1.5 × 20 cm) in hexane-ethyl acetate (2:1), this process permitting separation of excess α-ketoester as a forerun. The resulting product (0.274 g) was a mixture of carbazone isomers *(40)* by TLC (silica gel; hexanes-EtOAc (2:1) Rf=0.3 and 0.5. This was dissolved in ethanol (2 ml) and treated with sodium cyanoborohydride (27 mg; 0.52 mmol). After 4 hours, the reaction mixture was treated as described in Example 10 giving crude product as a colorless oil. Purification on silica gel (Baker; 1.5 × 20 cm) in hexanes — EtOAc (5:1) gave azapeptide *41* (0.181 g; 0.279 mmol; 36%) as a 1:1 mixture of diastereomers. TLC (silica gel; hexanes — EtOAc, 2:1; 2 developments) Rf=0.25, 0.30. MS m/e 652 ($M^+$). NMR ($CDCl_3$) 1.42 (18H, s); 1.7—2.8 (12H, m); 3.0—3.5 (6H, m); 4.2—4.6 (2H, m); 4.8—5.0 (1H, broad); 5.08, 5.12 (2H, 25); 7.1—7.2 (5H, bs); 7.25 (5H, s).

Example 34

$N^{\alpha}$-(1-Carbobenzyloxy-3-phenylpropyl)-α-azalysyl-(L)-proline *(42)*

A solution of azapeptide ester *41* (0.181 g; 0.279 mmol) in trifluoroacetic acid (4 ml) was allowed to stand for 4 hours at room temperature. The mixture was evaporated to dryness, and the resulting light brown residue was purified by chromatography DOWEX$^R$ 50W—X4 resin (10 g). Elution with $H_2O$-pyridine (50:1) gave pure azapeptide *42* (84.3 mg, 0.170 mmol; 61%) as a white foam. TLC (silica gel; EPAW 10:5:1:3) Rf=0.5. NMR (DMSO-$d_6$; 300 MHz) 1.4—2.0 (10H, m); 2.5—2.9 (2H, m); 3.1—3.7 (7H, m); 4.0—4.1 (1H, m); 5.09, 5.11 (2H, 2 × S); 7.1—7.3 (5H, m); 7.40 (5H, s). IR ($CHCl_3$): 3600—2500, 1730, 1630 $cm^{-1}$.

Example 35

$N^{\alpha}$-(1-Carboxy-3-phenylpropyl)-α-azalysyl-(L)-proline *(43)*

Azapeptide *42* (34.3 mg; 0.069 mmol) was combined with ethyl acetate (1.5 ml) and acetic acid (0.5 ml) and the resulting solution was exposed to 1 atm. of hydrogen, using 10% Pd(C) (50 mg) as catalyst. After 4 hours, the catalyst was removed by filtration, and the resulting product (14.5 mg) was purified by chromatography on a column of LH-20 (in methanol). The product *43* (9.4 mg; .023 mmol; 33%) showed a single spot on TLC (silica gel; EPAW 10:5:1:3) Rf=0.35. NMR (DMSO-d6)=1.6—2.0 (10H, m); 2.4—2.6 (2H, m); 2.8 (2H, bs); 3.0—3.5 (6H, m); 4.4 (1H, bs); 7.1—7.3 (5H, m).

Example 36

$N^{\alpha}$-(1-Carbobenzyloxy-5-phthalimidopentyl)-$N^{\delta}$-t-Boc-α-azalysyl-(L)-proline-t-butyl ester

Using the procedure described for azapeptide *14* in Example 10, carbazide *10* (0.142 g; 0.356 mmol) and benzyl 6-phthalimido-2-hexanoate (0.130 g; 0.356 mmol) were used to prepare azapeptide *44* in 50% yield (0.133 mg; 0.177 mmol) after flash chromatography on silica gel (1.5 × 22 cm; hexanes-EtAc, 1:1). TLC (silica gel, hexanes-EtAc, 1:1, 2 developments) showed 2 diastereomers, Rf 0.45, 0.50 (1:1). MS m/e 749 ($M^+$). IR ($CHCl_3$) 1775, 1730 (shoulder), 1710, 1645 $cm^{-1}$. NMR ($CDCl_3$) 1.42 (18H, s); 1.3—2.2 (14H, m); 3.0—3.8 (9H, m); 4.3—4.5 (2H, broad); 5.10, 5.13 (2H, 2 × S); 7.27 (5H, s); 7.7—7.9 (4H, m).

Example 37

$N^{\alpha}$-(1-Carboxyl-5-phthalimidopentyl)-$N^{\delta}$-t-Boc-α-azalysyl-(L)-proline-t-butyl ester *(45)*

A solution of azapeptide *44* in ethyl acetate (3 ml) containing 3 drops of glacial acetic acid was hydrogenated at 1 atm. for 18 hours with 10% Pd(C) (60 mg). The resulting azapeptide *45* (89.2 mg; 0.135 mmol; 94%) was purified by TLC (silica gel; EPAW; 20:5:1:1) Rf=0.75. MS m/e 803 ($M^+$, bistrimethylsilyl), NMR ($CDCl_3$); 1.43 (18H, m); 1.4—2.0 (14H, m); 3.0—3.8 (9H, m); 4.3—4.5 (1H, m); 5.0—5.3 (1H, broad); 7.2—7.4 (1H, broad); 7.7—7.9 (4H, m).

Example 38

$N^{\alpha}$-(1-Carboxy-5-phthalidimidopentyl)-α-azalysyl-(L)-proline *(46)*

A solution of azapeptide *45* (89.2 mg; 0.135 mmol) in trifluoroacetic acid (3 ml) was allowed to stand for 2 hours. Solvent was removed, and the light-brown residue was chromatographed on DOWEX$^R$ 50W—X4 (7.5 g). Elution with $H_2O$-pyr (50:1) gave pure azapeptide *46* (61.6 mg; 0.122 mmol; 91%) as a white foam. TLC silica gel; EPAW, 10:5:1:3) Rf=0.25. NMR ($CD_3OD$); 1.3—2.2 (14H, m); 2.9—3.1 (2H, m); 3.4—3.8 (7H, m); 4.3—4.6 (1H, m); 7.80 (4H, s).

Example 39

$N^{\alpha}$-(5-Amino-1-carboxypentyl)-α-azalysyl-(L)-proline *(47)*

To a solution of azapeptide *46* (55.6 mg; 0.110 mmol) in methanol (1 ml) was added anhydrous hydrazine (12 mg, 0.378 mmol). The solution was stirred for 18 hours and then filtered to remove the white precipitate. The residue, after evaporation, was chromatographed on DOWEX$^R$ 50W—X4 (8 g), using $H_2O$-pyr (50:1) as eluant. The product *(47)* (35.9 mg, 0.0923 mmol; 84%) was a white powder. TLC (silica gel; $H_2O$-BuOH-AcOH, 1:1:1) Rf=0.2, 0.4. MS m/e 661 (tetratrimethylsilyl). NMR (DMSO-d6)=1.3—1.8 (12H, m); 2.6—2.8 (2H, m); 3.0—3.6 (8H, m).

Example 40

N-(α-Azaalanyl)-(L)-1,2,3,4-tetrahydro-3-isoquinolinecarboxylic acid *t*-butyl ester *(48)*

(L)-1,2,3,4-Tetrahydro-3-isoquinolinecarboxylic acid, [prepared by the procedure of Yamada and Kunieda *Chem. Pharm. Bull., 15,* 490—8 (1967)] is converted to the corresponding *t*-butyl ester using the procedure of Taschner, et. al. [*Liebigs. Ann. Chem. 646,* 134—6 (1961)]. A solution of the ester and one equivalent of triethylamine in methylene chloride is addd to a slight excess of phosgene (12.5% solution in benzene). The resulting mixture is filtered and evaporated and the resulting residue is dissolved in methylene chloride and added with ice cooling to a solution of methylhydrazine (1 equiv.) and triethylamine (1 equiv.) in methylene chloride. The mixture is stirred for several hours, then filtered and evaporated. The resulting crude product is purified by chromatography on silica gel using methylene chloride-methanol mixtures as eluant.

Example 41

N-[N-(1-Carboethoxy-3-phenylpropyl)-α-azaalanyl]-(L)-1,2,3,4-tetrahydro-3-isoquinolinecarboxylic acid t-butyl ester *(49)*

Using the procedure described in Example 10, carbazide *(48)* and ethyl 4-phenyl-2-oxobutanoate are used to prepare azapeptide *(49)*, which is purified by chromatography on silica gel with methylene chloride-methanol mixtures as eluant.

Example 42

N-[N-(1-Carboethoxy-3-phenylpropyl)-α-azaalanyl]-(L)-1,2,3,4-tetrahydro-3-isoquinolinecarboxylic acid *(50)*

A solution of azapeptide *(49)* in trifluoroacetic acid is allowed to stand for 4 hours at room temperature.

Solvent is removed and the residue is chromatographed on DOWEX[R] 50W—X4 resin, using $H_2O$-pyr (50:1) as eluant, providing the product, after freeze-drying, as a white foam.

### Example 43

N-[N-(1-Carboxy-3-phenylpropyl)-α-azaalanyl]-L-1,2,3,4-tetrahydro-3-isoquinolinecarboxylic acid *(51)*

A solution of azapeptide *(50)* in $H_2O$-$CH_3OH$ (1:1) is treated with sodium hydroxide (2.5 equivalents) for 24 hours. The mixture is placed onto a column of DOWEX[R] 50W—X4. Elution with $H_2O$-pyr (50:1) gives the product, after freeze-drying, as a white foam.

### Example 44

$N^α$-(1-Carboethoxy-3-phenylpropyl)-$N^δ$-t-Boc-α-azalysyl)(L)-proline t-butyl ester *(52R, 52S)*

A solution of carbazide *10* (0.593 g; 1.48 mmol) and ethyl 4-phenyl-2-oxobutanoate (0.336 g; 1.63 mmol) in dry THF (7.5 ml) was heated at reflux for 16 hours. The solvent was removed by evaporation and absolute ethanol (5 ml) and sodium cyanoborohydride (93 mg; 1.48 mmol) were added. The solution was stirred for 4 hours, then treated as described in Example 10, giving the crude product as a colorless oil. TLC (silica gel; 1:1 hexanes-EtOAc) showed a mixture (1:1) of diastereomers, Rf—0.25, 0.30. Purification by column chromatography on silica gel (1:1 hexanes-EtOAc) (yield = 0.755 g; 1.28 mmol; 87%), followed by careful separation of the diastereomers on silica gel (2:1) hexanes-EtOAc) provided the following:

1) *52* More mobile diastereomer (184 mg). MS m/e 591 ($M^+$ +1) Analysis: Calculated for $C_{31}H_{50}N_4O_7$: C, 63.02; H, 8.53; N, 9.49. Found: C, 63.17; H, 8.60; N, 9.12. NMR ($CDCl_3$); 1.28 (3H, t, J=6); 1.43 (9H, S); 1.48 (9H, S); 1.6—2.1 (10H, m); 2.2—2.3 (2H, m); 2.67 (2H, t, J=8); 3.0—3.5 (7H, m); 4.17 (2H, q, J=7); 4.22 (1H, t, J=7); 4.94 (1H, broad S); 5.05 (1H, broad S); 7.1—7.3 (5H, m).

2) Mixed fractions (70 mg).

3) *52R*; Less mobile diastereomer (327 mg). MS: m/e 591 ($M^+$ +1) Analysis: Found: C, 62.59; H, 8.53; N 9.21. NMR ($CDCl_3$); 1.28 (3H, t, J=7); 1.46 (18H, S); 1.6—2.1 (10H, m); 2.2—2.3 (2H, m); 2.6—2.8 (2H, m); 3.1—3.3 (4H, m); 3.4—3.5 (3H, m); 4.17 (2H, q, J=7); 4.40 (1H, t, J=7); 4.9—5.0 (1H, broad); 7.2—7.4 (5H, m).

### Example 45

N-(1-Carboethoxy-3-phenylpropyl)-α-azalysyl-(L)-proline dihydrochloride *(53R, 53S)*

A solution of 52S (more-mobile diastereomer; 108 mg) in ice-cold concentrated hydrochloric acid (6 ml) was stirred for 18 hours at 5°C. The solution was diluted with $H_2O$ (25 ml) and evaporated giving *53S* as a white solid. (89 mg) TLC (silica gel; 70:30:6:4 $CHCl_3$:$CH_3OH$:$H_2O$:HOAc) Rf=0.5. NMR ($D_2O$): 1.18 (3H, t, J=7); 1.5—2.0 (10H, m); 2.1—2.3 (2H, m); 2.5—2.7 (2H, m); 2.9—3.1 (2H, m); 3.1—3.6 (5H, m); 4.09 (2H, q, J=7); 4.3—4.4 (1H, m); 7.1—7.3 (5H, m). Starting from *52R* (less-mobile diastereomer; 111 mg) there was obtained, by the same procedure, *53R* (90 mg) as a white solid. TLC (silica gel; 70:30:6:4 $CHCl_3$:$CH_3OH$:$H_2O$:HOAc) Rf=0.5. NMR ($D_2O$): 1.24 (3H, t, J=7); 1.5—2.1 (10H, m); 2.2—2.4 (2H, m); 2.6—2.8 (2H, m); 2.9—3.1 (2H. m); 3.2—3.7 (5H, m); 4.12 (2H, q, J=7); 4.6—4.7 (1H, m); 7.2—7.4 (5H, m).

### Example 46

N-(1-Carboxy-3-phenylpropyl)-α-azalysyl-(L)-proline dihydrochloride *(54R, 54S)*

A solution of *53S* (89 mg) in aqueous sodium hydroxide solution (0.25N; 3 ml) was stirred for 18 hours, then neutralized by addition of hydrochloric acid (0.50N). The product was freeze-dried, then purified by ion-exchange on DOWEX[R] 50W—X4. Elution with 25:1 $H_2O$:pyr and freeze-drying provided *54S* (free base) (65 mg; 0.16 mmol; 87%) as a white foam. This was further provided by chromatography on silica gel (70:30:6:4 $CHCl_3$:$CH_3OH$:$H_2O$:HOAc) and reconcentrated from hydrochloric acid (6N; 5 ml), giving *54S* (5 mg) as the dihydrochloric salt. TLC (silica gel, 70:30:6:4. CMWA) Rf=0.35. Analysis: Calculated for $C_{20}H_{28}N_4O_5$.2HCl.7/2 $H_2O$: C, 44.44; H, 6.90; N, 10.37. Found: C, 44.44; H, 6.78; N, 10.15. NMR ($D_2O$): 1.5—2.1 (10H, m); 2.2—2.4 (2H, m); 2.6—2.8 (2H, m); 2.9—3.1 (2H, m); 3.3—3.6 (5H, m); 4.3—4.45 (1H, m); 7.2—7.4 (5H, m).

Starting from *53R* (90 mg), there was obtained, using the same procedure, *54R* (54 mg) as the dihydrochloride salt. NMR ($D_2O$): 1.5—2.1 (10H, m); 2.1—2.3 (2H, m); 2.6—2.8 (2H, m); 3.2—3.7 (5H, m); 4.4—4.45 (1H, m); 7.2—7.4 (5H, m). TLC (silica gel: 70:30:6:4 CMWA) Rf=0.35.

### Example 47

N-(1-Carboethoxy-3-phenylpropyl)-α-azaalanyl-(L)-proline ethyl ester hydrochloride *(55)*

An ice-cold solution of N-(1-carboethoxy-3-phenylpropyl)-α-azaalanyl-(L)-proline *t*-butyl ester *(16S)* in absolute ethanol was saturated with hydrogen chloride. After one hour, the solution was evaporated to dryness and the product purified by chromatography on LH-20 (in methanol).

### Example 48

Ethyl 4-phenyl-2-hydrazinobutanone trifluoroacetate *(56)*

A solution of ethyl 4-phenyl-2-oxobutanoate (2.06 g; 10 mmol) and *t*-butylcarbazate (1.32 g; 10 mmol) in THF (50 ml) can be warmed at reflux for 8 hours. Solvent can be evaporated and the residue taken up in anhydrous ethanol (25 ml). Sodium cyanoborohydride (0.31 g; 5 mmol) can be added, and the solution stirred for 4 hrs. The residue, after evaporation, can be stirred with EtOAc (50 ml) and hydrochloric acid

(0.5N: 20 ml) for 30 minutes. The aqueous layer can be neutralized by addition of $K_2CO_3$ and extracted with EtOAc (3 × 25 ml). The combined organic portions can be washed with $H_2O$ and brine and dried ($MgSO_4$). The residue, upon evaporation, can be purified by chromatography on silica gel, affording N-(1-carboethoxy-3-phenylpropyl)-N'-BOC-hydrazine. An ice-cold solution of the above hydrazine in trifluoroacetic acid can be allowed to stand for 1 hr. Removal of solvent can provide hydrazine *(56)* as the trifluoroacetic acid salt.

Example 49

(4R)-4-Carboethoxy-2-(2-hydroxyphenyl)thiazolidine *(57)*

The procedure of M. Schubert (*J. Biol. Chem., 114,* 341 (1936)) was used to prepare *57* starting from L-cysteine ethyl ester and salicylaldehyde. Purification by silica gel chromatrography afforded *57* as a thick oil. TLC (silica gel; 3:1 cyclohexane-EtOAc) showed a mixture of 2 isomers, Rf=0.4, 0.5. IR (film): 3300 (sharp), 3000, 1740, 1430 $cm^{-1}$. NMR ($CCl_4$): 1.28 (3H, t, J=7); 2.9—3.5 (2H, m); 3.8—4.4 (3H, m); 5.50, 5.80 (1H, 2 × broad s); 6.5—7.3 (4H, m).

Example 50

N-[N-(1-Carboxy-3-phenylpropyl)-α-azaglycinyl]-4(R)-carboethoxy-2-(2-hydroxyphenyl)thiazolidine *(58)*

An ice-cold solution of phosgene (1.1 M solution in toluene; 0.91 ml) was diluted with $CH_2Cl_2$ (3 ml) and then a solution of *56* (0.34 g, 1.0 mmol) and triethylamine (0.28 ml; 2.0 mmol) in $CH_2Cl_2$ (3 ml) was added slowly with cooling (ice-bath). Then a solution of *57* (0.25 g; 1.0 mmol) and triethyl amine (0.14 ml; 1.0 mmol) in $CH_2Cl_2$ (3 ml) was added and stirring was contined at room temperature for 6 hours. The reaction mixture was diluted with EtAc (25 ml) and extracted with $H_2O$ (2 × 10 ml) and brine, and dried ($MgSO_4$). The residue after evaporation was subjected to chromatography on silica gel, allowing isolation of *58*.

Example 51

N-[(N-(1-Carboxy-3-phenylpropyl)-α-azaglycyl]-4-(R)-carboxy-2-(2-hydroxyphenyl)thiazolidine *(59)*

The above diester (Example 50) can be dissolved in aqueous sodium hydroxide (2N; 5 ml) and stirred for 1 hr. The resulting solution can be acidified with dilute hydrochloric acid and the product *(59)* isolated by ion-exchange chromatography on DOWEX 50W—X4 resin.

Example 52

Improved Procedure for: N-t-BOC-4-Aminobutyraldehyde carbobenzoxyhydrazone *(7)*

A solution of 4-aminobutyraldehyde diethyl acetal (9.33 g) and di-*t*-butyldicarbonate (13.3 g) in chloroform (120 ml) was allowed to stir for 2 hrs. The mixture was concentrated and to the residue were added THF (110 ml) and hydrochloric acid (0.5 N; 120 ml). The mixture was stirred vigorously for 1 hr, then cooled in an ice-bath and diluted with ether (100 ml). Sodium hydroxide solution (1N) was added until the aqueous layer was slightly basic (pH=8). The layers were separated and the aqueous layer extracted with ether (3 × 100 ml). The combined ether positions were washed with $H_2O$ and brine, and dried ($K_2CO_3$). Evaporation gave crude N-*t*-BOC-2-pyrrolidinol *(6)* as a colorless oil (20 g). This material was combined with carbobenzoyhydrazine (6.64 g) and THF (60 ml). The resulting solution was warmed at reflux for 3 hours, then allowed to stir for 20 hours at room temperature. Evaporation of solvent and recrystallization of the resulting solid from hexanes-EtOc gave *7* (8.4 g; 43%), m.p. 99—104°C. TLC (silica gel, EtOAc) showed a mixture (1:1) of isomers Rf=0.50, 0.55. Analysis: Calculated for $C_{17}H_{26}N_3O_4$: C, 60.70; H, 7.79; N, 12.49; Found: C, 61.09; H, 7.69; N, 12.45. NMR ($CDCl_3$): 1.43, 1.47 (9H, 2xs); 1.8—2.1 (2H, m); 2.2—2.5 (1H, m); 3.0—3.5 (3H, m); 4.8—5.0 (1H, m); 5.18, 5.27 (2H, 2xs); 7.3 (5H, s).

Example 53

N-(t-Boc-4-aminobutyl)-N'-CBZ-hydrazine *(8)*

To a solution of carbazone *7* (6.0 g) in THF (35 ml) and ethanol (20 ml) was added sodium cyanoborohydride (1.1 g). The mixture was cooled (ice-bath) and a solution of anhydrous HCl in ethanol (a saturated solution diluted 4 times with ethanol) was added slowly. After 6 ml had been added, TLC showed no carbazone remaining. The mixture was concentrated and then diluted with EtOAc (125 ml) and aqueous hydrochloric acid (0.5N; 30 ml). The mixture was stirred vigorously for 15 minutes and then the aqueous layer was neutralized by addition of $K_2CO_3$ and extracted with EtOAc (3 × 50 ml). The combined organic portions were washed with $H_2O$ and brine and dried ($MgSO_4$). The residue after evaporation was purified by column chromatography on silica gel and then recrystallized from hexanes-EtOAc, giving *8* (2.7 g; 8.2 mmol; 46%) m.p. 73—74°C.

Example 54

N-t-BOC-α-azalysyl-L-proline-t-butyl ester *(10)*

A solution of carbazide *8* (1.50 g; 4.45 mmol) and carbamoyl chloride (prepared as described in Example 1; 6.7 mmol, crude) in dry THF (7.5 ml) containing triethylamine (0.66 ml; 4.50 mmol) was warmed at reflux for 8 hrs. The residue, after evaporation was slurred in EtOAc and filtered. Purification by column chromatography on silica gel (hexanes-EtOAc) gave $NN^{\alpha}$-CBZ-$N^{\delta}$-*t*-BOC-α-azalysyl-(L)-proline-*t*-

butyl ester (2.29 g, 4.29 mmol; 96%) as a colorless oil. TLC (silica gel, 1:1 hexanes-EtOAc) Rf=0.35. Analysis: Calculated for $C_{27}H_{42}N_4O_7$: C, 60.66; H, 7.92; N, 10.48. Found: C, 61.15; H, 8.33; N, 10.04. NMR ($CDCl_3$); 1.43 (18H, s); 1.5—1.7 (4H, m); 1.75—2.3 (4H, m); 3.1—3.4 (3H, m); 3.4—3.6 (4H, m); 4.3 (1H, broad); 4.8 (1H, broad); 5.22 (2H, broad s); 7.4 (5H, broad s). A solution of this material in absolute ethanol (20 ml) was hydrogenated with 10% pd(C) (100 mg) for 3 hrs (1 atm). Filtration of the catalyst and removal of solvent gave carbazide *10* as a colorless oil. TLC (silica gel, 10:1 $CH_2Cl_2$:$CH_3OH$) Rf=0.7.

A portion of this material (0.10 g) was purified by silica gel chromatography (10:1 ether:methanol). Analysis: Calculated for $C_{19}H_{36}N_4O_5$: C, 56.97; H, 9.06; N, 13.99. Found: C, 57.00; H, 9.29; N, 14.06.

## Example 55

Improved procedure for: N-(1-Carboethoxy-3-phenylpropyl)-α-azaalanyl-(2)-proline *(17S)*

Azapeptide *16S* (0.180 g; 0.416 mmol) was combined with concentrated hydrochloric acid (5 ml) and the resulting solution was stirred overnight at 5°C. After dilution with $H_2O$ (25 ml), solvent was removed, giving *17S* as a light-yellow oil. This was purified by chromatography on silica gel (85:30:5:1) ($CHCl_3$—$CH_3OH$—$H_2O$—HOAc), then reconcentrated from dilute hydrochloric acid, giving the product (as the hydrochloride) as an oil (0.141 g; 0.341 mmol; 82%). TLC (silica gel, 20:5:1:1 EPAW) Rf=0.65.

## Example 56

N-(1-Carboxy-3-phenylpropyl)-α-azaalanyl-(L)-proline *(19S)*

A solution of azapeptide *16S* (more mobile diastereomer; (0.335 mg; 0.774 mmol) in trifluoroacetic acid (5 ml) was allowed to stand for 3 hours. The residue after evaporation was combined with sodium hydroxide (1N; 3 ml) and THF (0.5 ml) and the resulting solution was stirred under nitrogen for 18 hrs. After neutralization with dilute hydrochloric acid, the solution was placed onto a column of DOWEX 50W—X4 ion-exchange resin (5 g). The product was eluted with 20:1 $H_2O$-pyr and freeze-dried to a white foam (0.215 g; 0.1616 mmol; 80%). A portion of this material (0.052 g) was purified by chromatography on silica gel (83:30:5:1 $CHCl_3$—$CH_3OH$—$H_2O$:HOAc), affording pure product (0.031 g). The disodium salt (0.025 g) precipitated from $CH_2Cl_2$; m.p. 220—225 (dec). TLC (silica gel; 10:5:1:3 EPAW) Rf=0.45. Analysis: Calculated for $C_{17}H_{23}N_3O_5$. 2Na: C, 51.65; H, 5.82; N, 10.63. Found: C, 51.54; H, 5.79; N, 10.28. MS (for diacid): m/e 403 (monotrimethylsilyl) of $M^+$—$H_2O$). NMR ($CDCl_3$): 1.7—2.3 (6H, m); 2.6—2.8 (2H, m); 3.00 (3H, s); 3.5—3.6 (3H, m); 4.3—4.5 (1H, m); 7.1—7.3 (5H, m); 7.4—8.0 (2H, broad).

## Example 57

Additional Compounds of Formula I

Using the procedures and intermediates illustrated in the Examples above, it is possible to synthesize additional compounds of Formula I as shown in Table III. These syntheses make use of the t-butyl esters of amino acids of formula

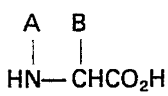

from Table I and known keto acids or esters listed in Table II.

TABLE I

Amino acids of the formula:

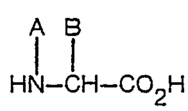

| | |
|---|---|
| (a) | 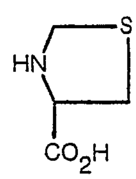 |
| (b) | 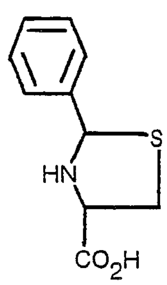 |

TABLE I

(c)

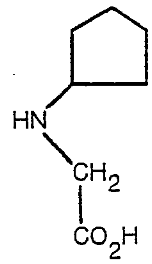

(d)

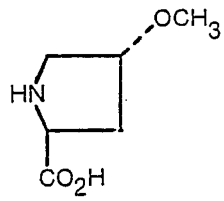

(e)

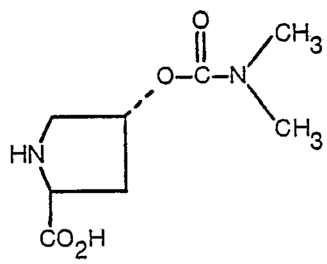

(f)

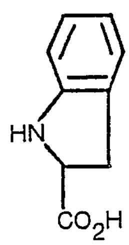

(g)

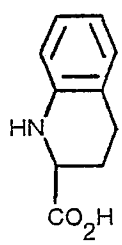

TABLE II

Keto acids and Esters of the formula:

$$R_1-CO-CO_2R$$

VI

(h) 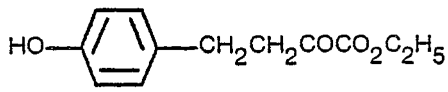

(i) 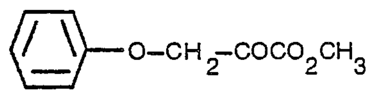

(j) 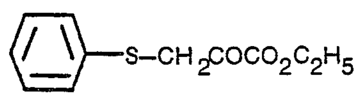

(k) 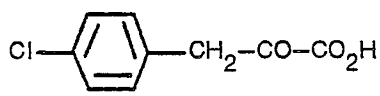

(l) 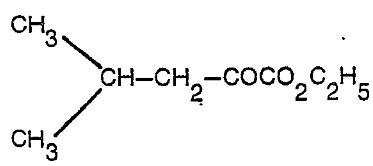

(m) 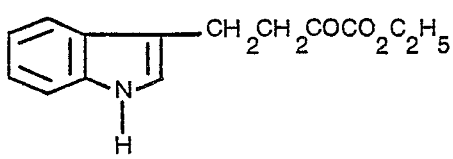

TABLE III

Additional compounds of Formula I:

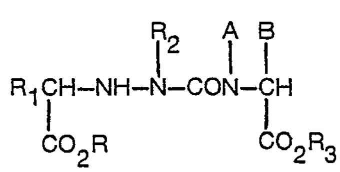

---

(n) 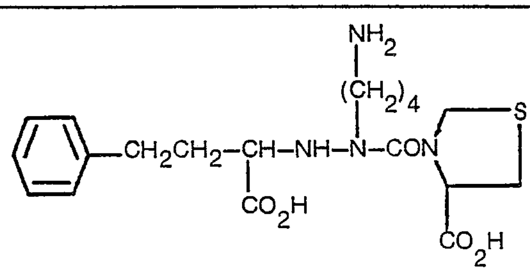

TABLE III

(o)

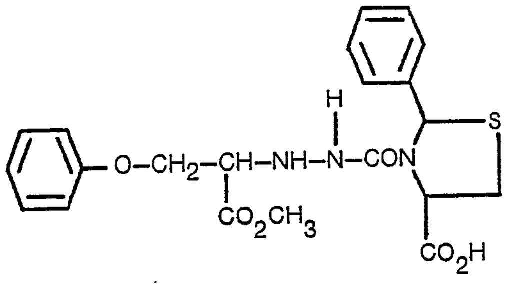

(p)

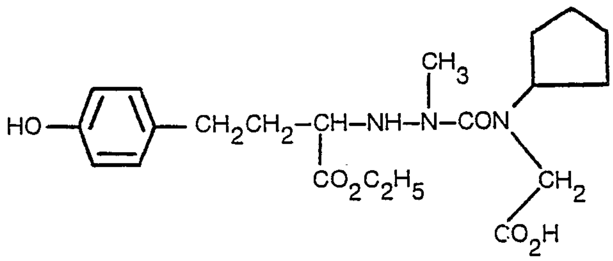

(q)

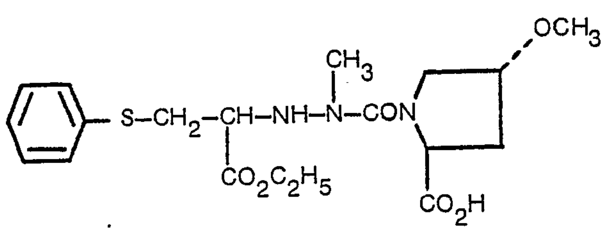

(r)

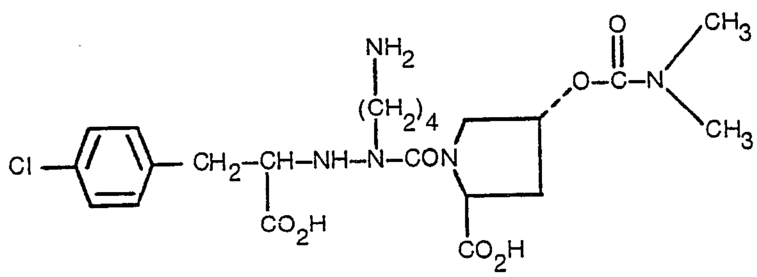

(s)

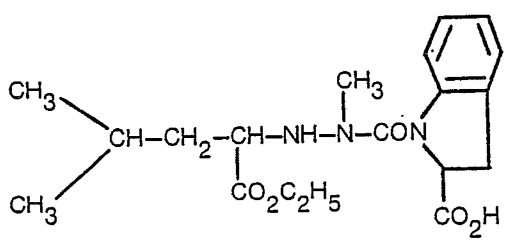

(t)

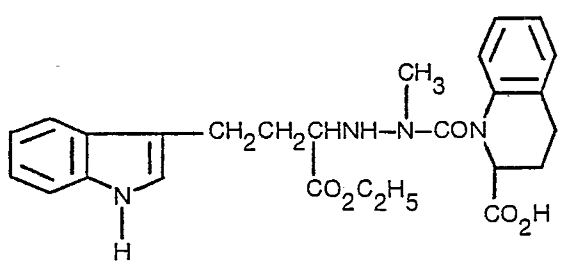

**Claims for the Contracting States: BE CH DE FR GB IT LI LU NL SE**

1. A compound having the formula

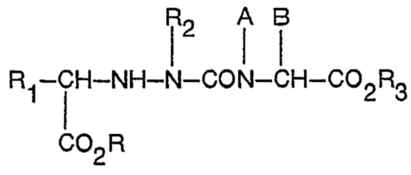

wherein:

R and $R_3$ are independently hydrogen, $C_{1-6}$ alkyl, ar-$C_{1-6}$-alkyl;

$R_1$ is aralkyl, heteroaralkyl, substituted aralkyl and substituted heteroaralkyl wherein the alkyl groups contain 1—6 carbon atoms and wherein the substituents in the aryl and heteroaryl groups can each independently be amino, halo, amino-$C_{1-6}$-alkyl, hydroxy, $C_{1-6}$ alkoxy, or $C_{1-6}$ alkyl; alkyl of 1—8 carbon atoms and alkyl of 1—8 carbon atoms substituted by amino, acylamino wherein acyl represents $C_{1-6}$ alkanoyl or aroyl groups, heteroarylamino, aryloxy, heteroaryloxy, arylthio, heteroarylthio or hydroxyl;

$R_2$ is hydrogen, $C_{1-6}$alkyl, amino-$C_{1-6}$-alkyl, ar-$C_{1-6}$-alkyl;

A is cycloalkyl of 5—7 carbon atoms, aryl, heteroaryl;

B is hydrogen, $C_{1-6}$ alkyl; or

A and B can be joined together to form ring structures which include N—$CHCO_2R_3$ and which have the formulae

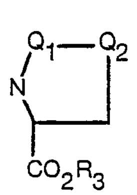

and

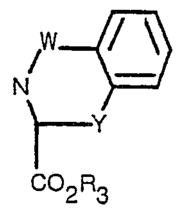

wherein

$R_3$ is as defined above;

$Q_1$ and $Q_2$, taken together, are $CH_2CH_2$, $CH_2$—S, $CHR_4S$, $CH_2$—CH—$OR_5$ wherein $R_4$ is aryl or aryl substituted by hydroxyl, amino, $C_{1-6}$ alkyl, amino, halo, or $C_{1-6}$ alkoxy; and

$R_5$ is hydrogen, $C_{1-6}$ alkyl, aryl, ar-$C_{1-6}$-alkyl, or $CONR_6R_7$ wherein $R_6$ and $R_7$ can each independently be hydrogen, $C_{1-6}$ alkyl, ar-$C_{1-6}$-alkyl;

W is a bond, $CH_2$;

Y is a bond, $CH_2$, $CH_2CH_2$;

the term "aryl" representing phenyl, naphthyl or biphenyl; the term "heteroaryl" representing pyridyl, thienyl, furyl, indolyl, benzthienyl, imidazolyl and thiazolyl; and the terms "aralkyl" and "heteroaralkyl" representing the corresponding radicals having 1—6 carbon atoms in the alkyl portion;

and, the pharmaceutically acceptable salts thereof.

2. A compound of Claim 1 wherein the asymmetric carbon atoms are in the L-amino acid configuration.

3. N-(1-carboxy-3-phenylpropyl)-α-azaalanyl-L-proline.

4. N-[1(S)-carboxy-3-phenylpropyl)-α-azaalanyl-L-proline.

5. N-(1-ethoxycarbonyl-3-phenylpropyl)-α-azaalanyl-L-proline.

6. N-[1(S)-carboethoxy-3-phenylpropyl)-α-azaalanyl-L-proline.

7. N-(1-carboethoxy-3-phenylpropyl)-α-azaalanyl-L-proline ethyl ester.

8. N-[1(S)-carboethoxy-3-phenylpropyl)-α-azaalanyl-L-proline ethyl ester.

9. $N^{\alpha}$-(1-carboxy-3-phenylpropyl)-α-azalysyl-L-proline.

10. $N^{\alpha}$-[(S)-carboxy-3-phenylpropyl)-α-azalysyl-L-proline.

11. A pharmaceutical composition useful in the treatment of hypertension comprising a pharmaceutically acceptable carrier and a pharmaceutically effective amount of a compound according to claim 1.

12. The composition of Claim 11 wherein said compound is a member of the group:

N-(1-carboxy-3-phenylpropyl)-α-azaalanyl-L-proline;
N-[1(S)-carboxy-3-phenylpropyl)-α-azaalanyl-L-proline.
N-(1-carboethoxy-3-phenylpropyl)-α-azaalanyl-L-proline;
N-[1-(S)-carboethoxy-3-phenylpropyl)-α-azaalanyl-L-proline;
N-(1-carboethoxy-3-phenylpropyl)-α-azaalanyl-L-proline ethyl ester;
N-[1(S)-carboethoxy-3-phenylpropyl)-α-azaalanyl-L-proline ethyl ester;
$N^{\alpha}$-(1-carboxy-3-phenylpropyl)-α-azalysyl-L-proline; and
$N^{\alpha}$-[(S)-carboxy-3-phenylpropyl)-α-azalysyl-L-proline.

13. The composition of Claim 11 wherein said composition also contains another antihypertensive and/or diuretic compound selected from hydrochlorothiazide, timolol, methyl dopa, the pivaloyloxyethyl ester

of methyl dopa, indacrinone and variable ratios of its enantiomers, (−)-4-{3-{-[2-(hydroxycyclohexyl)-ethyl]-4-oxo-2-thiazolidinyl}propyl}benzoic acid, as well as mixtures and combinations thereof.

14. A process for preparing compounds according to Claim 1 which process comprises reductively condensing a protected semicarbazide derivative having the formula:

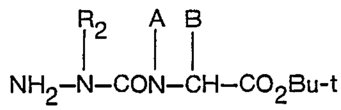

V

wherein $R_2$, A and B are as defined above, with a keto acid or keto ester having the formula:

$$R_1COCO_2R \quad VI$$

wherein R and $R_1$ are as defined above, to yield compounds of Formula I wherein $R_3$ is t-butyl, and, if desired, removing the t-butyl ester group yielding the compound wherein $R_3$ is hydrogen, and, for preparing compounds, wherein $R_3$ is not hydrogen, introducing $R_3$ using $R_3X$ wherein X is bromo or iodo, removing any remaining protecting groups and, if desired, separating the more biologically active diastereomer by chromatography or crystallization and, if further desired, preparing a pharmaceutically acceptable salt thereof by standard means.

**Claims for the Contracting State: AT**

1. A process for preparing compounds having the formula:

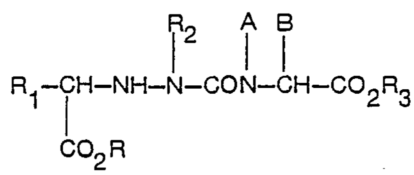

wherein:

R and $R_3$ are independently hydrogen, $C_{1-6}$ alkyl, ar-$C_{1-6}$-alkyl;

$R_1$ is aralkyl, heteroaralkyl, substituted aralkyl and substituted heteroaralkyl wherein the alkyl groups contain 1—6 carbon atoms and wherein the substituents in the aryl and heteroaryl groups can each independently be amino, halo, amino-$C_{1-6}$-alkyl, hydroxy, $C_{1-6}$ alkoxy, or $C_{1-6}$ alkyl; alkyl of 1—8 carbon atoms and alkyl of 1—8 carbon atoms substituted by amino, acylamino wherein acyl represents $C_{1-6}$ alkanoyl or aroyl groups, heteroarylamino, aryloxy, heteroaryloxy, arylthio, heteroarylthio or hydroxyl;

$R_2$ is hydrogen, $C_{1-6}$alkyl, amino-$C_{1-6}$-alkyl, ar-$C_{1-6}$-alkyl;

A is cycloalkyl of 5—7 carbon atoms, aryl, heteroaryl;

B is hydrogen, $C_{1-6}$ alkyl; or

A and B can be joined together to form ring structures which include N—$CHCO_2R_3$ and which have the formulae

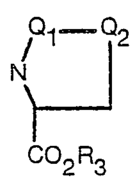

and

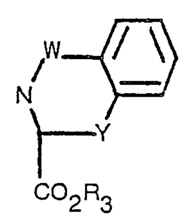

wherein

$R_3$ is as defined above;

$Q_1$ and $Q_2$, taken together, are $CH_2CH_2$, $CH_2$—S, $CHR_4S$, $CH_2$—CH—$OR_5$ wherein $R_4$ is aryl or aryl substituted by hydroxyl, amino, $C_{1-6}$ alkyl, amino, halo, or $C_{1-6}$ alkoxy; and

$R_5$ is hydrogen, $C_{1-6}$ alkyl, aryl, ar-$C_{1-6}$-alkyl, or $CONR_6R_7$ wherein $R_6$ and $R_7$ can each independently be hydrogen, $C_{1-6}$ alkyl, ar-$C_{1-6}$-alkyl;

W is a bond, $CH_2$;

Y is a bond, $CH_2$, $CH_2CH_2$;

the term "aryl" representing phenyl, naphthyl or biphenyl; the term "heteroaryl" representing pyridyl, thienyl, furyl, indolyl, benzthienyl, imidazolyl and thiazolyl; and the terms "aralkyl" and "heteroaralkyl" representing the corresponding radicals having 1—6 carbon atoms in the alkyl portion;

and, the pharmaceutically acceptable salts thereof which process comprises: reductively condensing a protected semicarbazide derivative having the formula:

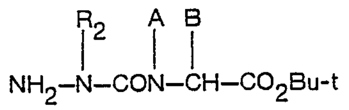

V

wherein $R_2$, A and B are as defined above, with a keto acid or keto ester having the formula:

$$R_1COCO_2R \qquad VI$$

wherein R and $R_1$ are as defined above, to yield compounds of Formula I wherein $R_3$ is t-butyl, and, if desired, removing the t-butyl ester group yielding the compound wherein $R_3$ is hydrogen, and, for preparing compounds, wherein $R_3$ is not hydrogen, introducing $R_3$ using $R_3X$ wherein X is bromo or iodo, removing any remaining protecting groups and, if desired, separating the more biologically active diastereomer by chromatography or crystallization and, if further desired, preparing a pharmaceutically acceptable salt thereof by standard means.

2. The process of Claim 1 for preparing compounds wherein the asymmetric carbon atoms are in the L-amino acid configuration.

3. The process of Claim 2 for preparing N-(1-carboxy-3-phenylpropyl)-α-azaalanyl-L-proline.

4. The process of Claim 2 for preparing N-[1(S)-carboxy-3-phenylpropyl)-α-azaalanyl-L-proline.

5. The process of Claim 2 for preparing N-(1-ethoxycarbonyl-3-phenylpropyl)-α-azaalanyl-L-proline.

6. The process of Claim 2 for preparing N-[1(S)-carboethoxy-3-phenylpropyl)-α-azaalanyl-L-proline.

7. The process of Claim 2 for preparing N-(1-carboethoxy-3-phenylpropyl)-α-azaalanyl-L-proline ethyl ester.

8. The process of Claim 2 for preparing N-[1(S)-carboethoxy-3-phenylpropyl)-α-azaalanyl-L-proline ethyl ester.

9. The process of Claim 2 for preparing $N^{α}$-(1-carboxy-3-phenylpropyl)-α-azalysyl-L-proline.

10. The process of Claim 2 for preparing $N^{α}$-[1(S)-carboxy-3-phenylpropyl]-α-azalysyl-L-proline.

**Patentansprüche für die Vertragsstaaten: BE CH DE FR GB IT LI LU NL SE**

1. Eine Verbindung mit der Formel

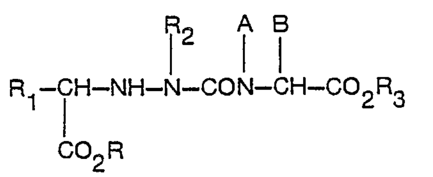

,

worin:

R und $R_3$ unabhängig voneinander Wasserstoff, $C_{1-6}$-Alkyl oder Ar-$C_{1-6}$-alkyl sind;

$R_1$ Aralkyl, Heteroaralkyl, substituiertes Aralkyl und substituiertes Heteroaralkyl, wobei die Alkylgruppen 1—6 Kohlenstoffatome enthalten und wobei die Substituenten in den Aryl- und Heteroarylgruppen jeweils unabhängig voneinander Amino, Halogen, Amino-$C_{1-6}$-alkyl, Hydroxy, $C_{1-6}$-Alkoxy oder $C_{1-6}$-Alkyl sein können; Alkyl mit 1—8 Kohlenstoffatomen oder Alkyl mit 1—8 Kohlenstoffatomen, das durch Amino, Acylamino, wobei Acyl $C_{1-6}$-Alkanoyl- oder Aroylgruppen bedeutet, Heteroarylamino, Aryloxy, Heteroaryloxy, Arylthio, Heteroarylthio oder Hydroxyl substituiert ist, darstellt;

$R_2$ Wasserstoff, $C_{1-6}$-Alkyl, Amino-$C_{1-6}$-alkyl oder Ar-$C_{1-6}$-alkyl ist;

A Cycloalkyl mit 5—7 Kohlenstoffatomen, Aryl oder Heteroaryl ist;

B Wasserstoff oder $C_{1-6}$-Alkyl ist; oder

A und B miteinander unter Bildung von Ringstrukturen verbunden sein können, welche N—$CHCO_2R_3$ umfassen und welche die Formeln

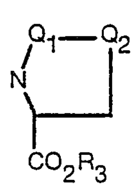

und

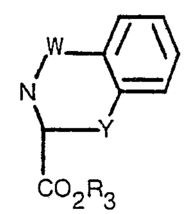

haben, worin

$R_3$ wie oben definiert ist;

$Q_1$ und $Q_2$ zusammengenommen $CH_2CH_2$, $CH_2$—S, $CHR_4S$, $CH_2$—CH—$OR_5$ sind, wobei $R_4$ Aryl oder

Aryl, das durch Hydroxyl, Amino, $C_{1-6}$-Alkyl, Amino, Halogen oder $C_{1-6}$-Alkoxy substituiert ist, bedeutet; und

$R_5$ Wasserstoff, $C_{1-6}$-Alkyl, Aryl, Ar-$C_{1-6}$-alkyl oder $CONR_6R_7$, wobei $R_6$ und $R_7$ jeweils unabhängig voneinander Wasserstoff, $C_{1-6}$-Alkyl oder Ar-$C_{1-6}$-alkyl sein können, bedeutet;

W eine Bindung oder $CH_2$ ist;

Y eine Bindung, $CH_2$ oder $CH_2CH_2$ ist; wobei der Ausdruck "Aryl" Phenyl, Naphthyl oder Biphenyl bedeutet; der Ausdruck "Heteroaryl" Pyridyl, Thienyl, Furyl, Indolyl, Benzthienyl, Imidazolyl oder Thiazolyl bedeutet; und die Ausdrücke "Aralkyl" und Heteroaralkyl" die entsprechenden Reste mit 1—6 Kohlenstoffatomen in dem Alkylabschnitt bedeuten;

und die pharmazeutisch annehmbaren Salze davon.

2. Eine Verbindung des Anspruchs 1, wobei die asymmetrischen Kohlenstoffatome in der L-Aminosäurekonfiguration vorliegen.

3. N-(1-Carboxy-3-phenylpropyl)-α-azaalanyl-L-prolin.

4. N-[1(S)-Carboxy-3-phenylpropyl)-α-azaalanyl-L-prolin.

5. N-(1-Ethoxycarbonyl-3-phenylpropyl)-α-azaalanyl-L-prolin.

6. N-[1(S)-Carboethoxy-3-phenylpropyl)-α-azaalanyl-L-prolin.

7. N-(1-Carboethoxy-3-phenylpropyl)-α-azaalanyl-L-prolinethylester.

8. N-[1(S)-Carboethoxy-3-phenylpropyl)-α-azaalanyl-L-prolinethylester.

9. $N^α$-(1-Carboxy-3-phenylpropyl)-α-azalysyl-L-prolin.

10. $N^α$-[1(S)-Carboxy-3-phenylpropyl]-α-azalysyl-L-prolin.

11. Eine pharmazeutische Zusammensetzung, die zur Behandlun g von Hypertension nützlich ist, enthaltend einen pharmazeutisch annehmbaren Träger und eine pharmazeutisch wirksame Menge einer Verbindung nach Anspruch 1.

12. Die Zusammensetzung des Anspruche 11, worin die genannte Verbindung ein Glied der Gruppe:
N-(1-Carboxy-3-phenylpropyl)-α-azaalanyl-L-prolin.
N-[1(S)-Carboxy-3-phenylpropyl)-α-azaalanyl-L-prolin.
N-(1-Carboethoxy-3-phenylpropyl)-α-azaalanyl-L-prolin.
N-[1(S)-Carboethoxy-3-phenylpropyl)-α-azaalanyl-L-prolin.
N-(1-Carboethoxy-3-phenylpropyl)-α-azaalanyl-L-prolinethylester.
N-[1(S)-Carboethoxy-3-phenylpropyl)-α-azaalanyl-L-prolinethylester.
$N^α$-(1-Carboxy-3-phenylpropyl)-α-azalysyl-L-prolin; und
$N^α$-[1(S)-Carboxy-3-phenylpropyl]-α-azalysyl-L-prolin ist.

13. Die Zusammensetzung des Anspruchs 11, worin die genannte Zusammensetzung auch eine andere antihypertensive und/oder diuretische Verbindung enthält, die aus Hydrochlorothiazid, Timolol, Methyldopa, dem Pivaloyloxyethylester von Methyldopa, Indacrinon und veränderlichen Verhältnissen seiner Enantiomeren, (−)-4-{3-{[2-(Hydroxycyclohexyl)-ethyl]-4-oxo-2-thiazolidinyl}propyl}lbenzosäure, sowie Mischungen und Kombinationen davon ausgewählt ist.

14. Ein Verfahren zur Herstellung von Verbindungen nach Anspruch 1, welches Verfahren umfaßt:
die reduktive Kondensation eines geschützten Semicarbazidderivats mit der Formel:

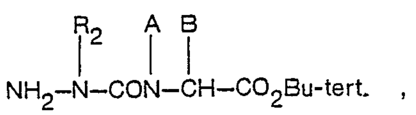 V

worin $R_2$, A und B wie oben definiert sind, mit einer Ketosäure oder einem Ketoester der Formel:

$$R_1COCO_2R \qquad VI$$

worin R und $R_1$ wie oben definiert sind, unter Bildung von Verbindungen der Formel I, worin $R_3$ tert.Butyl ist, und gewünschtenfalls die Entfernung der tert.Butylestergruppe unter Bildung der Verbindung, worin $R_3$ Wasserstoff ist, und zur Herstellung von Verbindungen, worin $R_3$ nicht Wasserstoff ist, die Einführung von $R_3$ unter Verwendung von $R_3X$, worin X Brom oder Iod ist,

die Entfernung irgendwelcher restlicher Schutzgruppen, und, gewünschtenfalls, die Abtrennung des biologisch wirksameren Diastereomers durch Chromatographie oder Kristallisation, und, falls weiterhin erwünscht, die Herstellung eines pharmazeutisch annehmbaren Salzes davon, durch übliche Mittel.

**Patentansprüche für den Vertragsstaat: AT**

1. Ein Verfahren zur Herstellung von Verbindungen der Formel

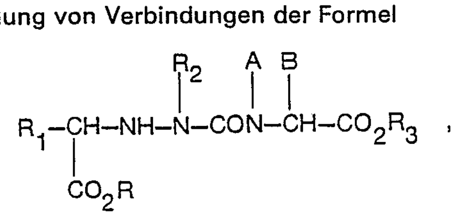

worin:

R und $R_3$ unabhängig voneinander Wasserstoff, $C_{1-6}$-Alkyl oder Ar-$C_{1-6}$-alkyl sind;

$R_1$ Aralkyl, Heteroaralkyl, substituiertes Aralkyl und substituiertes Heteroaralkyl, wobei die Alkylgruppen 1—6 Kohlenstoffatome enthalten und wobei die Substituenten in den Aryl- und Heteroarylgruppen jeweils unabhängig voneinander Amino, Halogen, Amino-$C_{1-6}$-alkyl, Hydroxy, $C_{1-6}$-Alkoxy oder $C_{1-6}$-Alkyl sein können; Alkyl mit 1—8 Kohlenstoffatomen oder Alkyl mit 1—8 Kohlenstoffatomen, das durch Amino, Acylamino, wobei Acyl $C_{1-6}$-Alkanoyl- oder Aroylgruppen bedeutet, Heteroarylamino, Aryloxy, Heteroaryloxy, Arylthio, Heteroarylthio oder Hydroxyl substituiert ist, darstellt;

$R_2$ Wasserstoff, $C_{1-6}$-Alkyl, Amino-$C_{1-6}$-alkyl oder Ar-$C_{1-6}$-alkyl ist;

A Cycloalkyl mit 5—7 Kohlenstoffatomen, Aryl oder Heteroaryl ist;

B Wasserstoff oder $C_{1-6}$-Alkyl ist; oder

A und B miteinander unter Bildung von Ringstrukturen verbunden sein können, welche N—$CHCO_2R_3$ umfassen und welche die Formeln

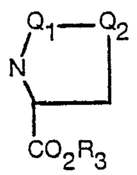

und

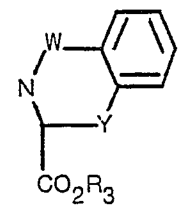

haben, worin

$R_3$ wie oben definiert ist;

$Q_1$ und $Q_2$ zusammengenommen $CH_2CH_2$, $CH_2$—S, $CHR_4S$, $CH_2$—CH—$OR_5$ sind, wobei $R_4$ Aryl oder Aryl, das durch Hydroxyl, Amino, $C_{1-6}$-Alkyl, Amino, Halogen oder $C_{1-6}$-Alkoxy substituiert ist, bedeutet; und

$R_5$ Wasserstoff, $C_{1-6}$-Alkyl, Aryl, Ar-$C_{1-6}$-alkyl oder $CONR_6R_7$, wobei $R_6$ und $R_7$ jeweils unabhängig voneinander Wasserstoff, $C_{1-6}$-Alkyl oder Ar-$C_{1-6}$-alkyl sein können, bedeutet;

W eine Bindung oder $CH_2$ ist;

Y eine Bindung, $CH_2$ oder $CH_2CH_2$ ist; wobei der Ausdruck "Aryl" Phenyl, Naphthyl oder Biphenyl bedeutet; der Ausdruck "Heteroaryl" Pyridyl, Thienyl, Furyl, Indolyl, Benzthienyl, Imidazolyl oder Thiazolyl bedeutet; und die Ausdrücke "Aralkyl" und Heteroaralkyl" die entsprechenden Reste mit 1—6 Kohlenstoffatomen in dem Alkylabschnitt bedeuten;

und den pharmazeutisch annehmbaren Salzen davon, welches Verfahren umfaßt:

die reduktive Kondensation eines geschützten Semicarbazidderivats mit der Formel:

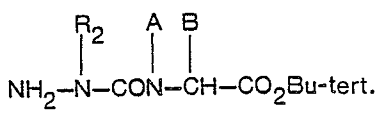 ,

V

worin $A_1$, A und B wie oben definiert sind, mit einer Ketosäure oder einem Ketoester der Formel:

$$R_1COCO_2R \qquad VI$$

worin R und $R_1$ wie oben definiert sind, unter Bildung von Verbindungen der Formel I, worin $R_3$ tert.Butyl ist, und gewünschtenfalls die Entfernung der tert.Butylestergruppe unter Bildung der Verbindung, worin $R_3$ Wasserstoff ist, und zur Herstellung von Verbindungen, worin $R_3$ nicht Wasserstoff ist, die Einführung von $R_3$ unter Verwendung von $R_3X$, worin X Brom oder Iod ist,

die Entfernung irgendwelcher restlicher Schutzgruppen, und, gewünschtenfalls, die Abtrennung des biologisch wirksameren Diastereomers durch Chromatographie oder Kristallisation, und, falls weiterhin erwünscht, die Herstellung eines pharmazeutisch annehmbaren Salzes davon, durch übliche Mittel.

2. Das Verfahren des Anspruchs 1 zur Herstellung von Verbindungen, worin die asymmetrischen Kohlenstoffatome in der L-Aminosäurekonfiguration vorliegen.

3. Das Verfahren des Anspruchs 2 zur Herstellung von N-(1-Carboxy-3-phenylpropyl)-α-azaalanyl-L-prolin.

4. Das Verfahren des Anspruchs 2 zur Herstellung von N-[1(S)-Carboxy-3-phenylpropyl]-α-azaalanyl-L-prolin.

5. Das Verfahren des Anspruchs 2 zur Herstellung von N-(1-Ethoxycarbonyl-3-phenylpropyl)-α-azaalanyl-L-prolin.

6. Das Verfahren des Anspruchs 2 zur Herstellung von N-[1(S)-Carboethoxy-3-phenylpropyl]-α-azaalanyl-L-prolin.

7. Das Verfahren des Anspruchs 2 zur Herstellung von N-(1-Carboethoxy-3-phenylpropyl)-α-azaalanyl-L-prolinethylester.

8. Dàs Verfahren des Anspruchs 2 zur Herstellung von N-[1(S)-Carboethoxy-3-phenylpropyl]-α-azaalanyl-L-prolinethylester.

9. Das Verfahren des Anspruchs 2 zur Herstellung von $N^{\alpha}$-(1-Carboxy-3-phenylpropyl)-α-azalysyl-L-prolin.

10. Das Verfahren des Anspruchs 2 zur Herstellung von $N^{\alpha}$-[1(S)-Carboxy-3-phenylpropyl]-α-azalysyl-L-prolin.

**Revendications pour les Etats contractants: BE CH DE FR GB IT LI LU NL SE**

1. Composé de formule

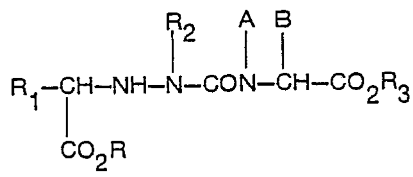

où

R et $R_3$ représentent indépendamment hydrogène, alcoyle en $C_1$ à $C_6$, aralcoyle en $C_1$ à $C_6$;

$R_1$ représente aralcoyle, hétéroaralcoyle, aralcoyle substitué et hétéroaralcoyle substitué où les groupes alcoyle contiennent de 1 à 6 atomes de carbone et où les substituants dans les groupes aryle et hétéroaryle peuvent être chacun indépendamment amino, halo, aminoalcoyle en $C_1$ à $C_6$, hydroxy, alcoxy en $C_1$ à $C_6$, ou alcoyle en $C_1$ à $C_6$; alcoyle en $C_1$ à $C_8$ et alcoyle en $C_1$ à $C_8$ substitué par amino, acylamino où acyle représente des groupes alcanoyle en $C_1$ à $C_6$ ou aroyle, hétéroarylamino, aryloxy, hétéroaryloxy, arylthio, hétéroarylthio ou hydroxyle;

$R_2$ représente hydrogène, alcoyle en $C_1$ à $C_6$, amino-alcoyle en $C_1$ à $C_6$, aralcoyle en $C_1$ à $C_6$;

A représente cycloalcoyle en $C_5$ à $C_7$, aryle, hétéroaryle;

B représente hydrogène alcoyle en $C_1$ à $C_6$; ou

A et B peuvent être réunis pour former des structures cycliques qui comprenent N—$CHCO_2$ $R_3$ et qui sont de formules

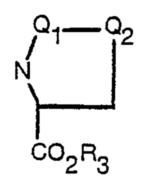

et

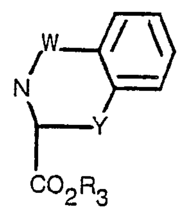

où

$R_3$ est tel que défini ci-dessus;

$Q_1$ et $Q_2$, pris ensemble, sont $CH_2CH_2$, $CH_2$—S, $CHR_4S$, $CH_2$—CH—$OR_5$ où

$R_4$ est un aryle ou un aryle substitué par un hydroxyle, un amino, un alcoyle en $C_1$ à $C_6$, un amino, un halo, ou un alcoxy en $C_1$ à $C_6$; et

$R_5$ est un hydrogène, un alcoyle en $C_1$ à $C_6$, aryle, ar-alcoyle en $C_1$ à $C_6$, ou $CONR_6R_7$ où $R_6$ et $R_7$ peuvent être chacun indépendamment hydrogène, alcoyle en $C_1$ à $C_6$, ar-alcoyle en $C_1$ à $C_6$;

W est une liaison, $CH_2$;

Y est une liaison, $CH_2$, $CH_2CH_2$;

le terme "aryle" représentant phényle, naphtyle, ou biphényle; le terme "hétéroaryle" représentant pyridyle, thiényle, furyle, indolyle, benzthiényle, imidazolyle et thiazolyle; et les termes "aralcoyle" et "hétéroaralcoyle" représentant les radicaux correspondants ayant de 1 à 6 atomes de carbone dans la fraction alcoyle;

et ses sels pharmaceutiquement acceptables.

2. Composé de la revendication 1 où les atomes de carbone asymétriques sont dans la configuration L d'acide aminé.

3. N-(1-carboxy-3-phénylpropyl)-α-azaalanyl-L-proline.

4. N-[1(S)-carboxy-3-phénylpropyl]-α-azaalanyl-L-proline.

5. N-(1-éthoxycarbonyl-3-phénylpropyl]-α-azaalanyl-L-proline.

6. N-[1(S)-carboéthoxy-3-phénylpropyl]-α-azaalanyl-L-proline.

7. N-(1-carboéthoxy-3-phénylpropyl]-α-azaalanyl-L-proline-éthyl-ester.

8. N-[1(S)-carboéthoxy-3-phénylpropyl]-α-azaalanyl-L-proline-éthyl-ester.

9. $N^{\alpha}$-(1-carboxy-3-phénylpropyl)-α-azalysyl-L-proline.

10. $N^{\alpha}$-[1(S)-carboxy-3-phénylpropyl]-α-azalysyl-L-proline.

11. Composition pharmaceutique utile dans le traitement de l'hypertension comprenant un support

pharmaceutiquement acceptable et une quantité pharmaceutiquement efficace d'un composé selon la revendication 1.

12. Composition de la revendication 11 où ledit composé est un membre du groupe:
N-(1-carboxy-3-phénylpropyl)-α-azaalanyl-L-proline.
N-[1(S)-carboxy-3-phénylpropyl]-α-azaalanyl-L-proline.
N-(1-carboéthoxy-3-phénylpropyl]-α-azaalanyl-L-proline.
N-[1(S)-carboéthoxy-3-phénylpropyl]-α-azaalanyl-L-proline.
N-(1-carboéthoxy-3-phénylpropyl]-α-azaalanyl-L-proline-éthyl-ester.
N-[1(S)-carboéthoxy-3-phénylpropyl]-α-azaalanyl-L-proline-éthyl-ester.
$N^{\alpha}$-(1-carboxy-3-phénylpropyl)-α-azalysyl-L-proline.
$N^{\alpha}$-[1(S)-carboxy-3-phénylpropyl]-α-azalysyl-L-proline.

13. Composition de la revendication 11 où ladite composition contient également un autre composé antihypertenseur et/ou diurétique choisi entre l'hydrochlorothiazide, le timolol, la méthyl-dopa, le pivaloyloxyéthyl-ester de méthyldopa, l'indacrinone et des rapports variables de ses énantiomères, l'acide (−)-4-{3-{-[2-(hydroxycyclohexyl)-éthyl]-4-oxo-2-thiazolidinyl}propyl}benzoïque, ainsi que leurs mélanges et combinaisons.

14. Procédé de préparation de composés selon la revendication 1, procédé dans lequel:
on condense de façon réductrice un dérivé de semicarbazide protégé de formule:

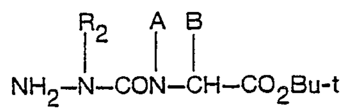
V

où $R_2$, A et B sont tels que définis ci-dessus, avec un céto-acide ou céto-ester de formule:

$$R_1COCO_2R \qquad VI$$

où R et $R_1$ sont tels que définis ci-dessus, pour donner des composés de formule I où $R_3$ est un t-butyle, et, si on le désire, on enlève le groupe t-butyl-ester, donnant le composé où $R_3$ est un hydrogène, et, pour préparer des composés où $R_3$ n'est pas un hydrogène, on introduit $R_3$ en utilisant $R_3$ X où X est un bromo ou iodo, on enlève les groupes protecteurs éventuellement restants, et, si on le désire, on sépare le diastéréomère le plus actif biologiquement par chromatographie ou cristallisation et, si on le désire encore, on prépare un de ses sels pharmaceutiquement acceptables par des moyens standard.

**Revendications pour l'Etat contractant: AT**

1. Procédé de préparation de composés de formule

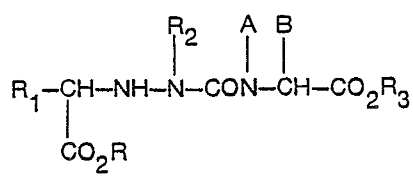

où

R et $R_3$ représentent indépendamment hydrogène, alcoyle en $C_1$ à $C_6$, aralcoyle en $C_1$ à $C_6$;

$R_1$ représente aralcoyle, hétéroaralcoyle, aralcoyle substitué et hétéroaralcoyle substitué où les groupes alcoyle contiennent de 1 à 6 atomes de carbone et où les substituants dans les groupes aryle et hétéroaryle peuvent être chacun indépendamment amino, halo, aminoalcoyle en $C_1$ à $C_6$, hydroxy, alcoxy en $C_1$ à $C_6$, ou alcoyle en $C_1$ à $C_6$; alcoyle en $C_1$ à $C_8$ et alcoyle en $C_1$ à $C_8$ substitué par amino, acylamino où acyle représente des groupes alcanoyle en $C_1$ à $C_6$ ou aroyle, hétéroarylamino, aryloxy, hétéroaryloxy, arylthio, hétéroarylthio ou hydroxyle;

$R_2$ représente hydrogène, alcoyle en $C_1$ à $C_6$, amino-alcoyle en $C_1$ à $C_6$, aralcoyle en $C_1$ à $C_6$;

A représente cycloalcoyle en $C_5$ à $C_7$, aryle, hétéroaryle;

B représente hydrogène alcoyle en $C_1$ à $C_6$; ou

A et B peuvent être réunis pour former des structures cycliques qui comprennent N—$CHCO_2$ $R_3$ et qui sont de formules

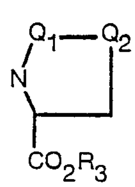
et
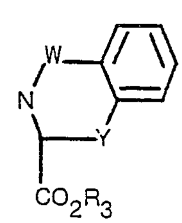

où

$R_3$ est tel que défini ci-dessus;

$Q_1$ et $Q_2$, pris ensemble, sont $CH_2CH_2$, $CH_2$—S, $CHR_4S$, $CH_2$—CH—$OR_5$ où

$R_4$ est un aryle ou un aryle substitué par un hydroxyle, un amino, un alcoyle en $C_1$ à $C_6$, un amino, un halo, ou un alcoxy en $C_1$ à $C_6$; et

$R_5$ est un hydrogène, un alcoyle en $C_1$ à $C_6$, aryle, ar-alcoyle en $C_1$ à $C_6$, ou $CONR_6R_7$ où $R_6$ et $R_7$ peuvent être chacun indépendamment hydrogène, alcoyle en $C_1$ à $C_6$, ar-alcoyle en $C_1$ à $C_6$;

W est une liaison, $CH_2$;

Y est une liaison, $CH_2$, $CH_2CH_2$;

le terme "aryle" représentant phényle, naphtyle, ou biphényle; le terme "hétéroaryle" représentant pyridyle, thiényle, furyle, indolyle, benzthiényle, imidazolyle et thiazolyle; et les termes "aralcoyle" et "hétéroaralcoyle" représentant les radicaux correspondants ayant de 1 à 6 atomes de carbone dans la fraction alcoyle;

et de leurs sels pharmaceutiquement acceptables, dans lequel: on condense de façon réductrice un dérivé de semicarbazide protégé de formule:

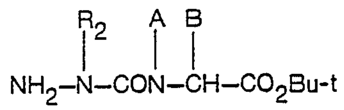

V

où $R_2$, A et B sont tels que définis ci-dessus, avec un céto-acide ou céto-ester de formule:

$$R_1COCO_2R \qquad VI$$

où R et $R_1$ sont tels que définis ci-dessus, pour donner des composés de formule I où $R_3$ est un t-butyle, et, si on le désire, on enlève le groupe t-butyl-ester, donnant le composé où $R_3$ est un hydrogène, et, pour préparer des composés où $R_3$ n'est pas un hydrogène, on introduit $R_3$ en utilisant $R_3X$ où X est un bromo ou iodo, on enlève les groupes protecteurs éventuellement restants, et, si on le désire, on sépare le diastéréomère le plus actif biologiquement par chromatographie ou cristallisation et, si on le désire encore, on prépare un de ses sels pharmaceutiquement acceptables par des moyens standard.

2. Procédé de la revendication 1 pour préparer des composés où les atomes de carbone asymétriques sont dans la configuration L d'acide aminé.

3. Procédé de la revendication 2 pour préparer la N-(1-carboxy-3-phénylpropyl)-α-azaalanyl-L-proline.

4. Procédé de la revendication 2 pour préparer la N-[1(S)-carboxy-3-phénylpropyl)-α-azaalanyl-L-proline.

5. Procédé de la revendication 2 pour préparer la N-(1-éthoxycarbonyl-3-phénylpropyl)-α-azaalanyl-L-proline.

6. Procédé de la revendication 2 pour préparer la N-[1(S)-carboéthoxy-3-phénylpropyl)-α-azaalanyl-L-proline.

7. Procédé de la revendication 2 pour préparer la N-(1-carboéthoxy-3-phénylpropyl)-α-azaalanyl-L-proline-éthyl-ester.

8. Procédé de la revendication 2 pour préparer la N-[1(S)-carboéthoxy-3-phénylpropyl)-α-azaalanyl-L-proline-éthyl-ester.

9. Procédé de la revendication 2 pour préparer la $N^{\alpha}$-(1-carboxy-3-phénylpropyl)-α-azalysyl-L-proline.

10. Procédé de la revendication 2 pour préparer la $N^{\alpha}$-[1(S)-carboxy-3-phénylpropyl)-α-azalysyl-L-proline.